# EUROPEAN PATENT SPECIFICATION

(11) **EP 3 989 875 B1**
(45) Date of publication and mention of the grant of the patent: **27.05.2026**
(21) Application number: 20830714.0
(22) Date of filing: 25.06.2020
(51) Int. Cl.: A61F 2/02, A61L 24/10, C07K 14/76, A61K 9/14

(54) **REACTIVE DRY POWDERED HEMOSTATIC MATERIALS COMPRISING A NUCLEOPHILE AND A MULTIFUNCTIONAL MODIFIED POLYETHYLENE GLYCOL BASED CROSSLINKING AGENT**
REAKTIVE TROCKENE PULVERFÖRMIGE HÄMOSTATISCHE MATERIALIEN MIT EINEM NUKLEOPHIL UND EINEM MULTIFUNKTIONELLEN MODIFIZIERTEN VERNETZUNGSMITTEL AUF POLYETHYLENGLYKOLBASIS
MATÉRIAUX HÉMOSTATIQUES PULVÉRULENTS SECS RÉACTIFS COMPRENANT UN NUCLÉOPHILE ET UN AGENT DE RÉTICULATION À BASE DE POLYÉTHYLÈNE GLYCOL MODIFIÉ MULTIFONCTIONNEL

(30) Priority: 26.06.2019 US 201962867118 P
(43) Date of publication of application: 04.05.2022
(73) Proprietor: Davol Inc., Warwick, RI 02886 (US)
(72) Inventor: GREENAWALT, Keith, Milton, MA 02186 (US); STRICKLER, Frederick H., Jr., Boston, MA 02109 (US)
(74) Representative: Hoffmann Eitle
(86) International application number: PCT/US2020/039660
(87) International publication number: WO 2020/264188

(56) References cited:
- US-A1- 2013 096 063
- US-A1- 2015 306 277
- US-B1- 6 177 095
- US-B2- 6 576 263
- US-B2- 9 114 172
- US-E- R E38 158

## Description

### TECHNICAL FIELD

Compositions and methods related to powdered hemostats that crosslink during and/or after application to a bleeding site are generally described.

### BACKGROUND

Existing powdered hemostats such as degradable starch microspheres (DSMs) or oxidized regenerative cellulose (ORC) and flowable hemostats such as liquid thrombin or fibrin glue can suffer from poor tissue adherence and may not be sufficiently effective when used for certain bleeding/wound sites. This may be due to a lack of absorbency, insufficient tissue adherence and/or cohesivity at the wound site to resist being washed away in the case of powdered hemostats. Additionally, typical conventional hemostats do not form a strong hydrogel network. This can create a need for aggressive or prolonged manual compression to keep the products in place after application to a bleeding site, making it challenging for surgeons to continue to operate in the same area. Accordingly, improved hemostatic compositions and methods would be desirable.

US 2013/0096063 A1 describes a hemostatic composition comprising a biocompatible polymer in particulate form suitable for use in hemostasis, and one hydrophilic polymeric component comprising reactive groups.

US RE38,158 E describes an adhesive composition for bonding or sealing tissue in vivo that is formed from a two-component mixture which includes a first part of a protein in an aqueous buffer and a second part of a water-compatible or water-soluble bifunctional crosslinking agent.

### SUMMARY

In one aspect, disclosed and presently claimed is a dry, powdered, crosslinking hemostatic composition, comprising:
a first component comprising a difunctionalized polyalkylene oxide-based component of any of the forms: 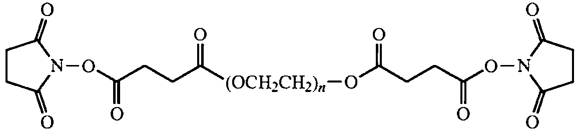 Poly(ethylene glycol) disuccinimidoyl succinate 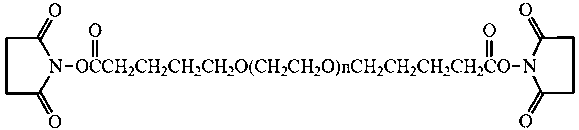 PEG disuccinimidyl valerate 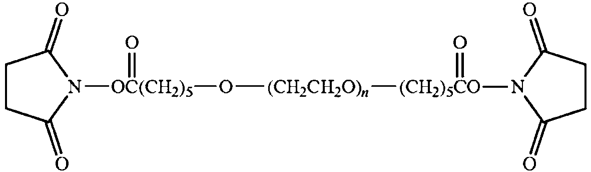 PEG disuccinimidyl hexanoate; and
a second component comprising:
   a protein that is capable of crosslinking with the first component; and
a crosslinking initiator that initiates crosslinking of the first component with the protein, wherein the crosslinking initiator comprises a base and/or a basic buffer, wherein the base and/or basic buffer comprises sodium bicarbonate and/or a base that does not include amine functionality;
wherein upon exposure to an aqueous liquid, crosslinking is initiated to form a hemostatic hydrogel.

In another aspect the dry, powdered, crosslinking hemostatic composition disclosed and presently claimed is indicated for use in a method of controlling bleeding, the method comprising:
applying a crosslinkable dry powder composition to a bleeding/wound site, and
allowing the dry powder composition to crosslink into a hemostatic hydrogel upon exposure to the bleeding/wound site capable of stopping or reducing bleeding at the bleeding/wound site.

Other advantages and novel features of the present invention will become apparent from the following detailed description of various embodiments of the invention when considered in conjunction with the accompanying figures.

### BRIEF DESCRIPTION OF THE DRAWINGS

Embodiments of the present invention will be described by way of example with reference to the accompanying figures, which are schematic and are not intended to be drawn to scale. In the figures, each identical or nearly identical component illustrated is typically represented by a single numeral. For purposes of clarity, not every component is labeled in every figure, nor is every component of each embodiment of the invention shown where illustration is not necessary to allow those of ordinary skill in the art to understand the invention. In the figures:
FIG. 1 shows, in accordance with certain embodiments, exemplary steps in a method for forming a hemostatic hydrogel with a dry powdered mixture;
FIG. 2A shows, in accordance with certain embodiments, the measured crosslink time for a dry powdered composition to achieve hemostasis as compared to other commercially available hemostats;
FIG. 2B shows, in accordance with certain embodiments, the percent of rebleeds after application of a dry powdered composition to a wound site as compared to other commercially available hemostats;
FIG. 3 shows, in accordance with certain embodiments, the wet field burst strength of a dry powdered composition as compared to a commercially available hemostat;
FIG. 4A shows, in accordance with certain embodiments, an image of a layer of blood;
FIG. 4B shows, in accordance with certain embodiments, an image of the application of a dry powdered hemostat to a layer of blood;
FIG. 4C shows, in accordance with certain embodiments, the formation of a hemostatic hydrogel on the layer of blood;
FIG. 5 shows, in accordance with certain embodiments, the effect of using various sources of albumin on the measured crosslink time of a dry powdered composition;
FIG. 6A shows, in accordance with certain embodiments, the effect of protein particle size on the burst strength of a dry powdered composition;
FIG. 6B shows, in accordance with certain embodiments, the effect of protein particle density on the burst strength of a dry powdered composition; and
FIG. 7 shows, in accordance with certain embodiments, a test fixture set up for measuring the burst strength of a hydrogel formed from a dry powdered composition.

### DETAILED DESCRIPTION

Compositions and methods related to powdered hemostats that crosslink during and/or after application to a bleeding site are generally described. In certain embodiments, upon exposure to an aqueous liquid, a dry powdered mixture of reactive hemostat components may crosslink to form a hemostatic hydrogel. Exposure to an aqueous liquid may be achieved as the dry, powdered, crosslinking compositions are applied to a bleeding/wound site comprising blood and/or other bodily fluids. In addition to, or instead of, use as a hemostatic material for controlling or stopping bleeding, in certain embodiments, compositions and methods described herein may be useful for a variety of other medical applications, such as postsurgical adhesion barriers, sealants, and wound dressings.

As used herein, the term "crosslink" refers to a chemical reaction between two or more similar or dissimilar polymers, copolymers, oligomers, and/or macromers that links the two or more similar or dissimilar polymers, copolymers, oligomers, or macromers via formation of at least one covalent bond and/or ionic bond, or a chain extension between one or more polymers, copolymers, oligomers, and/or macromers to provide a longer chain of the one or more polymers, copolymers, oligomers, and/or macromers via formation of at least one covalent bond and/or ionic bond.

Disclosed and presently claimed is a multi-component (e.g., two component, three component, etc.) composition is used. A first component comprises a difunctionalized polyalkylene oxide-based component as defined in the appended claims, and a second component comprises one or both of a protein (e.g., albumin) that is capable of crosslinking with the first component and a crosslinking initiator that initiates crosslinking of the first component with the protein. Crosslinking to form a hemostatic hydrogel occurs upon exposure of the composition to an aqueous liquid, such as at a bleeding/wound site. In some cases, the structural properties (e.g., particle size and/or particle density) of certain components of the dry powder composition may affect the time required for the dry powder composition to crosslink and form a hemostatic hydrogel or may affect the degree of crosslinking or both.

As disclosed and presently claimed, a two-component reactive dry powder composition (e.g., dry powder mixture) is used. In certain embodiments, the first component may comprise a first dry powder comprising a difunctionalized polyalkylene oxide-based component as defined in the appended claims, and a second component may comprise a second dry powder (a protein such as albumin) that is capable of crosslinking with the first dry powder. Upon exposure to an aqueous liquid an initiator is used to initiate crosslinking between the two different reactive powdered components, resulting in a crosslinked hemostatic hydrogel that is capable of stopping and/or reducing bleeding at the bleeding/wound site. Hemostatic precursors comprising crosslinking difunctionalized polyalkylene oxide-based components and protein (e.g. albumin) may help alleviate issues related to the need for manual compression by forming a hydrogel with tissue adherence, in certain embodiments.

According to some embodiments, the first component is in the form of a first powder (e.g., a first dry powder), and the second component is in the form of a second powder (e.g., a second dry powder). In certain embodiments, a two part crosslinking dry powder hemostatic formulation is provided. Further details regarding the form of the dry, powdered, hemostatic composition are discussed below.

In the present invention, the first component (e.g., first dry powder) comprises a difunctionalized polyalkylene oxide-based component of any of the forms:
PEG disuccinimidyl succinate (PEG(SS)2), a 2-arm crosslinker of the form: 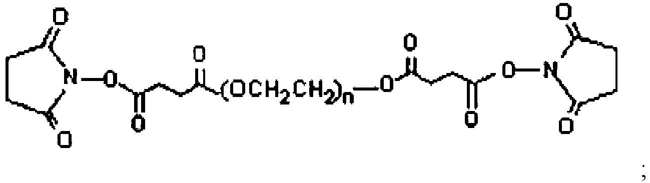
PEG disuccinimidyl valerate (PEG(SVA)2), a 2-arm crosslinking of the form: 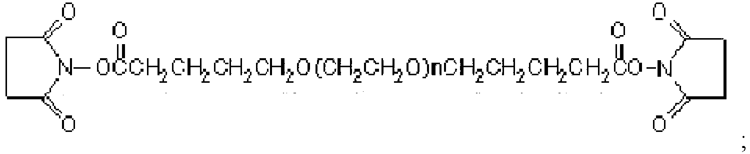
PEG disuccinimidyl hexanoate (PEG(SHA)2), a 2-arm crosslinker of the form:

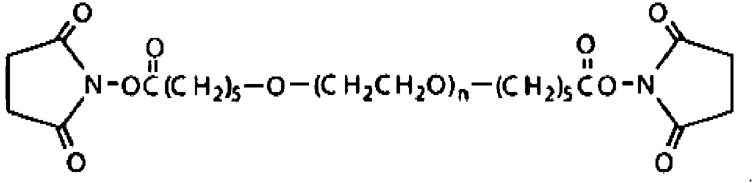

According to certain embodiments with a first component (e.g., first dry powder) comprising a difunctionalized polymeric composition according to the appended claims, the polymeric composition may have any of a variety of suitable weight average molecular weights. For example, in certain embodiments, the first component (e.g., first dry powder) may have a weight average molecular weight of greater than or equal to 1 kDa, greater than or equal to 5 kDa, greater than or equal to 10 kDa, greater than or equal to 15 kDa, greater than or equal to 20 kDa, or greater than or equal to 25 kDa. In certain embodiments, the polymeric composition may be a macromer having a weight average molecular weight of less than or equal to 30 kDa, less than or equal to 25 kDa, less than or equal to 20 kDa, less than or equal to 15 kDa, less than or equal to 10 kDa, or less than or equal to 5 kDa. Combinations of the above recited ranges are also possible (e.g., the first component comprises a difunctionalized polymeric composition with a weight average molecular weight of greater than or equal to 1 kDa and less than or equal to 30 kDa, or greater than or equal to 10 kDa and less than or equal to 15 kDa, and the like). In some embodiments, the weight average molecular weight of the first component (e.g., a first dry powder) comprising a difunctionalized polymeric composition is determined using size exclusion chromatography-multi-angle laser light scattering (SEC-MALLS).

According to certain embodiments, difunctionalized polymeric compositions may be prepared by any of a variety suitable synthetic methods known to those skilled in the art. For example, see, U.S. Patent 6,576,263, U.S. Patent RE38,827, and U.S. Patent RE38,158.

For example, the difunctionalized polymeric compositions may be prepared using known processes, procedures or synthetic methods such as the procedures reported in U.S. Patent 4,101,380 or 4,839,345, the procedure reported in International Application Ser. No. PCT/US90/02133 filed Apr. 19, 1990 or the procedure reported by Abuchowski et al., Cancer Biochem. Biophys., 7:175-186 (1984). Briefly, a polyalkylene oxide-based component (e.g., polyethylene glycol discussed below as exemplary) and a suitable acid anhydride are dissolved in a suitable polar organic solvent in the presence of base and refluxed for a period of time sufficient to form a polyethylene glycol diester diacid. The diester diacid is then reacted with a leaving group such as an N-hydroxy imide compound in a suitable polar organic solvent in the presence of dicyclohexylcarbodiimide or other condensing agents and stirred at room temperature to form the desired difunctional crosslinking agent.

All or some of the difunctionalized polymeric compositions may be purchased from commercial sources, including, but not limited to, NOF America Corporation and/or Laysan Bio, Inc. The difunctionalized polymeric compositions may also be readily synthetized by persons of ordinary skill in the chemical synthesis art in view the teaching and exemplary methods described herein for exemplary compositions, published literature, and the level of ordinary skill and knowledge of the skilled artisan.

In certain non-limiting embodiments, PEG(SS)2 can be synthesized by obtaining a linear PEG with an average weight average molecular weight of 3,350 Da, representing 75.7 oxyethylene repeat units. The linear PEG can be obtained, for example, from Dow Chemical Company. The linear PEG may be converted to PEG(SS)2 via a two-step synthesis, in some cases. For instance, the first step may comprise reacting the linear PEG with succinic anhydride to produce PEG(disuccinate), or PEG(SS). The second step may comprise reacting PEG(SS) with N-hydroxysuccinimide to produce PEG(SS)2, resulting in a white solid and a two arm crosslinker that possess two succinimidyl groups per molecule.

As disclosed and presently claimed, the second component comprises a protein. In certain cases, the protein comprises any of a variety of suitable albumins. For example, in some embodiments, the protein comprises serum albumin. The serum albumin may be, in some cases, human serum albumin (HSA) derived from donor blood, recombinant human albumin (rHA) derived from yeast, and/or animal sourced albumin (e.g., bovine serum albumin (BSA)). In certain non-limiting embodiments, for example, the protein may be Cohn analog culture grade BSA obtained from Proliant Biologicals. In some aspects, the recombinant human albumin may be Cellastim^{™} recombinant human albumin, Healthgen^{™} recombinant human albumin, or Optibumin^{™} recombinant human albumin.

According to some embodiments, the protein may comprise collagen or gelatin.

The above nucleophilic electrophilic crosslinking reactions are pH sensitive and are inhibited at acidic pH while being initiated by increasing the pH to neutral or basic values. The second component comprises an initiator (e.g., a crosslinking initiator) comprising a base or a basic buffer that may be used in combination with the reactive materials to initiate or facilitate crosslinking. The crosslinking initiator comprising a base and/or basic buffer facilitates the reaction between the leaving group in compositions of the formula described above and the amine group of a protein. In some cases, the base is sodium bicarbonate. According to certain embodiments, the basic crosslinking initiator is a base and/or basic buffer that does not include amine functionalities.

According to certain embodiments, the reaction between the leaving group and the amine group of the protein occurs at pH of greater than or equal to 7, and the crosslinking reaction in situ is made to occur (e.g. through addition to a base or basic buffer to one or both of the reactive components) at a pH of greater than or equal to 7, a pH of greater than or equal to 7.4, a pH of greater than or equal to 8, a pH of greater than or equal to 9, a pH of greater than or equal to 10.

According to certain embodiments, a dry powder hemostat comprises a mixture of a difunctionalized electrophilic polymeric first component (e.g., first dry powder such as PEG(SS)2) in any of a variety of suitable amounts in weight percent by mass in combination with a second component comprising a protein. For example, in some embodiments, the dry powder composition (e.g., dry powder mixture) comprises the first component (e.g., first dry powder) in an amount of greater than or equal to 15 wt.%, greater than or equal to 20 wt.%, greater than or equal to 25 wt.%, greater than or equal to 30 wt.%, or greater than or equal to 35 wt.% of the total mixture. In certain embodiments, the dry powder composition (e.g., dry powder mixture) comprises the first component (e.g., first dry powder) in an amount of less than or equal to 40 wt.%, less than or equal to 35 wt.%, less than or equal to 30 wt.%, less than or equal to 25 wt.%, or less than or equal to 20 wt.% of the total mixture. Combinations of the above recited ranges are also possible (e.g., the dry powder mixture comprises the first component in an amount of greater than or equal to 15 wt.% and less than or equal to 40 wt.% of the total mixture, the dry powder mixture comprises the first component in an amount of greater than or equal to 20 wt.% and less than or equal to 25 wt.% of the total mixture).

According to certain embodiments, the second component of the dry powder composition (e.g., dry powder mixture) may comprise the protein (e.g., albumin) in any of a variety of suitable amounts in weight percent by mass. For example, in certain embodiments, the dry powder composition (e.g., dry powder mixture) comprises the protein in an amount of greater than or equal to 20 wt.%, greater than or equal to 25 wt.%, greater than or equal to 30 wt.%, greater than or equal to 35 wt.%, greater than or equal to 40 wt.%, greater than or equal to 45 wt.%, greater than or equal to 50 wt.%, greater than or equal to 55 wt.%, or greater than or equal to 60 wt.% of the total mixture. In certain embodiments, the dry powder composition (e.g., dry powder mixture) comprises the protein in an amount of less than or equal to 65 wt.%, less than or equal to 60 wt.%, less than or equal to 55 wt.%, less than or equal to 50 wt.%, less than or equal to 45 wt.%, less than or equal to 40 wt.%, less than or equal to 35 wt.%, less than or equal to 30 wt.%, or less than or equal to 25 wt.% of the total mixture. Combinations of the above recited ranges are also possible (e.g., the dry powder mixture comprises the protein in an amount of greater than or equal to 20 wt.% and less than or equal to 65 wt.% of the total mixture, the dry powder mixture comprises the protein in an amount of greater than or equal to 40 wt.% and less than or equal to 50 wt.% of the total mixture). According to certain embodiments, the dry powder composition, when applied to blood, may require a lesser amount of the protein as compared to when the dry powder composition is applied to other media (e.g., saline) due to the presence of additional proteins (e.g., albumin) and/or cellular components in the blood.

According to some embodiments, the second component comprises a protein consisting essentially of particles having a certain particle size distribution and/or certain particle size. As used herein, the phrase "consisting essentially of particles having a certain particle size distribution" means that greater than or equal to 80 wt.% of the particles fall within the stated particle size range. In certain cases, greater than or equal to 90 wt.%, greater than or equal to 95 wt.%, greater than or equal to 98 wt.%, greater than or equal to 99 wt.%, or greater than or equal to 99.9 wt.% of the particles fall within the stated particle size range. Similarly, "consisting essentially of particles having a certain particle size" means that greater than or equal to 80 wt.% of the particles fall within a range that is ± 20% of the stated particle size. In certain cases, greater than or equal to 90 wt.%, greater than or equal to 95 wt.%, greater than or equal to 98 wt.%, greater than or equal to 99 wt.%, or greater than or equal to 99.9 wt.% fall within a range that is ± 20% of the stated particle size. Also similarly, "consisting essentially of particles not exceeding a certain particle size" or "consisting essentially of particles having at least a certain particle size" means that greater than or equal to 80 wt.% of the particles do not exceed, or have a size that is at least, respectively, the stated particle size. In certain cases, greater than or equal to 90 wt.%, greater than or equal to 95 wt.%, greater than or equal to 98 wt.%, greater than or equal to 99 wt.%, or greater than or equal to 99.9 wt.% of the particles do not exceed, or have a size that is at least, respectively, the stated particle size.

In certain embodiments, the protein particles are substantially spherical and the particle size is a maximum cross-sectional particle diameter. Other particle shapes, however, are also possible. Without wishing to be bound by theory, in some embodiments, the measured time it takes for the dry powdered composition to crosslink and/or the degree of crosslinking may depend on the particle size of the protein. Accordingly, it may be advantageous, in certain aspects, to employ a protein consisting essentially of particles within a certain particle size range in order to control the time it takes for the dry powdered composition to crosslink when applied to a bleeding/wound site and/or the extent of crosslinking, as is explained below in greater detail.

In certain embodiments, the protein particles may be separated by particle size (e.g., maximum particle diameter) using methods known to a person of ordinary skill in the art, such as using a sieve and/or filter to separate target particles above and below a certain sieve/filter cutoff size. In some embodiments the sieve-separated protein particle size may be further measured using spectroscopic techniques, such as dynamic light scattering (DLS), transmission electron microscopy (TEM), or scanning electron microscopy (SEM). In some aspects, the spectroscopic techniques may be used to supplement and/or confirm the particle size of the particles that have been separated using sieves and/or filters.

The protein particles may have any of a variety of suitable particle sizes. In certain embodiments, for example, the protein consists essentially of particles having a particle size of greater than or equal to 50 micrometers, greater than or equal to 100 micrometers, greater than or equal to 150 micrometers, greater than or equal to 200 micrometers, greater than or equal to 250 micrometers, greater than or equal to 300 micrometers, greater than or equal to 350 micrometers, greater than or equal to 400 micrometers, greater than or equal to 450 micrometers, or greater than or equal to 500 micrometers. In some embodiments, the protein consists essentially of particles having a particle size of less than or equal to 600 micrometers, less than or equal to 500 micrometers, less than or equal to 450 micrometers, less than or equal to 400 micrometers, less than or equal to 350 micrometers, less than or equal to 300 micrometers, less than or equal to 250 micrometers, less than or equal to 200 micrometers, less than or equal to 150 micrometers, or less than or equal to 100 micrometers. Combinations of the above recited ranges are also possible (e.g., the protein consists essentially of particles having a particle size of greater than or equal to 50 micrometers and less than or equal to 600 micrometers, the protein consists essentially of particles having a particle size of greater than or equal to 100 micrometers and less than or equal to 250 micrometers, etc.).

In certain embodiments, the second component comprises a protein that comprises a plurality of particles having a certain bulk or tapped particle density (e.g., tapped particle density). Without wishing to be bound by theory, in some embodiments, the measured time it takes for the dry powdered composition to crosslink and the degree of crosslinking may depend on the particle density (e.g., tapped particle density) of the protein. Accordingly, it may be advantageous, in certain aspects, to employ a composition comprising a protein with a certain particle density (e.g., tapped particle density) in order to control the time it takes for the dry powdered composition to crosslink or the degree of crosslinking when applied to a bleeding/wound site, as is explained below in greater detail.

In some embodiments, employing a composition comprising a protein with a certain particle density may have further advantages in addition to controlling the time it takes for the dry powdered composition to crosslink. In some aspects, for example, employing a composition comprising a protein with a certain particle density may affect the time it takes for the dry powdered composition to break the surface tension of blood, penetrate through the layer of blood, and adhere to the underlying tissue. **In** some embodiments, for example, a protein with a higher particle density may break the surface tension of blood, penetrate through the layer of blood, and adhere to the underlying tissue more quickly than a protein with a lower particle density.

According to some embodiments, the particle density of the protein may be measured using methods known to a person of ordinary skill in the art. For example, the particle densities referred to herein are determined using a tapped density method. Specifically, for the measurements made herein, a tapped density measurement is made as follows: the mass of the protein is measured using a standard analytical balance capable of reading up to 0.1 mg, for example, the mass of the protein may be measured by adding greater than a 6.0 ml volume of the protein to a calibrated 10 ml graduated cylinder that is capable or reading up to 0.1 ml (e.g., Pyrex No. 3022) that has been pre-tared on the analytical balance; the bottom of the graduated cylinder containing the protein is then repeatedly "tapped" against a flat surface in order to increase the packing density of the protein in the graduated cylinder until the volume of the protein does not change more than 0.1 mL between taps; and the tapped density is determined by dividing the measured mass by the measured volume.

In certain embodiments, the particle density of the protein can be controlled. In some embodiments, for example, the particle density of the protein may be changed by lyophilizing solutions of different concentrations of the protein. For example, in some embodiments, the particle density of the starting material of the protein may be determined as described above, and a solution of the protein is solubilized and lyophilized to provide a particle density that is different than the particle density of the starting material. In certain embodiments, the particle density of the protein after lyophilization is preferably lower than the particle density of the protein starting material. The final, post lyophilization density can be controlled at least in part by controlling the concentration of the protein in the solution that is lyophilized. In some embodiments, for example, more concentrated solutions lead to higher post-lyophilization densities as compared to less concentrated solutions. In some embodiments, the post-lyophilization density is lower than the particle density of the protein starting material. In certain other embodiments, the post-lyophilization density is greater than the particle density of the protein starting material. A post-lyophilization particle density that is greater than the starting material particle density may be obtained, in some embodiments, by lyophilizing solutions containing high concentrations of starting materials with low particle densities (e.g., less than 0.30 g/ml). In some embodiments, the particle density of the protein starting material may be increased by roller compacting and granulating the protein starting material. In certain embodiments, it may be advantageous to increase the particle density of the protein by roller compacting the second component comprising the protein and the crosslinking initiator (e.g., the base or basic buffer) together.

The protein particles may have any of a variety of suitable particle densities (e.g., tapped particle densities). For example, in certain embodiments, the protein comprises a plurality of particles having a particle density of greater than or equal to 0.30 g/ml, greater than or equal to 0.35 g/ml, greater than or equal to 0.40 g/ml, greater than or equal to 0.45 g/ml, greater than or equal to 0.50 g/ml, greater than or equal to 0.55 g/ml, greater than or equal to 0.60 g/ml, greater than or equal to 0.65 g/ml, greater than or equal to 0.70 g/ml, or greater than or equal to 0.75 g/ml. In some embodiments, the protein comprises a plurality of particles having a particle density of less than or equal to 0.80 g/ml, less than or equal to 0.75 g/ml, less than or equal to 0.70 g/ml, less than or equal to 0.65 g/ml, less than or equal to 0.60 g/ml, less than or equal to 0.50 g/ml, less than or equal to 0.45 g/ml, less than or equal to 0.40 g/ml, or less than or equal to 0.35 g/ml. Combinations of the above recited ranges are also possible (e.g., the protein comprises a plurality of particles having a particle density of greater than or equal to 0.30 g/ml and less than or equal to 0.80 g/ml, the protein comprises a plurality of particles having a particle density of greater than or equal to 0.35 g/ml and less than or equal to 0.45 g/ml).

In a specific, non-limiting embodiment, the dry powder composition comprises lyophilized bovine serum albumin with a tapped particle density greater than or equal to 0.60 g/ml and less than or equal to 0.70 g/ml. In another specific non-limiting embodiment, the dry powder composition comprises lyophilized bovine serum albumin with a particle density greater than or equal to 0.20 g/ml and less than or equal to 0.40 g/ml.

According to certain embodiments, the difunctionalized polymeric composition comprises a plurality of particles having any of a variety of suitable particle sizes and/or particle densities (e.g., tapped particle densities), which may be determined as described above in reference to the protein.

In some embodiments, the difunctionalized polymeric composition (e.g., PEG(SS)2, and/or any other of the first component electrophilic compositions described herein) comprises a plurality of particles having a particle size of greater than or equal to 10 micrometers, greater than or equal to 50 micrometers, greater than or equal to 100 micrometers, greater than or equal to 150 micrometers, greater than or equal to 200 micrometers, greater than or equal to 250 micrometers, greater than or equal to 300 micrometers, greater than or equal to 350 micrometers, greater than or equal to 400 micrometers, greater than or equal to 450 micrometers, greater than or equal to 500 micrometers, or greater than or equal to 550 micrometers. In certain embodiments, the electrophilic functionalized PEG difunctionalized polymeric composition comprises a plurality of particles having a particle size of less than or equal to 600 micrometers, less than or equal to 550 micrometers, less than or equal to 500 micrometers, less than or equal to 450 micrometers, less than or equal to 400 micrometers, less than or equal to 350 micrometers, less than or equal to 300 micrometers, less than or equal to 250 micrometers, less than or equal to 200 micrometers, less than or equal to 150 micrometers, less than or equal to 100 micrometers, or less than or equal to 50 micrometers. Combinations of the above recited ranges are also possible (e.g., the difunctionalized polymeric composition comprises a plurality of particles having a particle size greater than or equal to 10 micrometers and less than or equal to 600 micrometers, the difunctionalized polymeric composition comprises a plurality of particles having a particle size greater than or equal to 200 micrometers and less than or equal to 300 micrometers).

In certain embodiments, the difunctionalized polymeric composition comprises a plurality of particles having a particle density (e.g., tapped particle density) greater than or equal to 0.20 g/ml, greater than or equal to 0.25 g/ml, greater than or equal to 0.30 g/ml, greater than or equal to 0.35 g/ml, greater than or equal to 0.40 g/ml, greater than or equal to 0.45 g/ml, greater than or equal to 0.50 g/ml, or greater than or equal to 0.55 g/ml. In some embodiments, the difunctionalized polymeric composition comprises a plurality of particles having a particle density (e.g., tapped particle density) less than or equal to 0.60 g/ml, less than or equal to 0.55 g/ml, less than or equal to 0.50 g/ml, less than or equal to 0.45 g/ml, less than or equal to 0.40 g/ml, less than or equal to 0.35 g/ml, less than or equal to 0.30 g/ml, or less than or equal to 0.25 g/ml. Combinations of the above recited ranges are also possible (e.g., the difunctionalized polymeric composition comprises a plurality of particles having a particle density greater than or equal to 0.20 g/ml and less than or equal to 0.60 g/ml, the difunctionalized polymeric composition comprises a plurality of particles having a particle density greater than or equal to 0.25 g/ml and less than or equal to 0.35 g/ml).

According to some embodiments, the crosslinking initiator (e.g. a basic salt such as sodium bicarbonate, etc.) comprises a plurality of particles having any of a variety of suitable particle sizes and/or particle densities (e.g., tapped particle densities), which may be determined as described above in reference to the protein.

In certain embodiments, the crosslinking initiator comprises a plurality of particles having a particle size of greater than or equal to 20 micrometers, greater than or equal to 50 micrometers, greater than or equal to 100 micrometers, greater than or equal to 150 micrometers, greater than or equal to 200 micrometers, greater than or equal to 250 micrometers. In some embodiments, the crosslinking initiator comprises a plurality of particles having a particle size of less than or equal to 300 micrometers, less than or equal to 250 micrometers, less than or equal to 200 micrometers, less than or equal to 150 micrometers, less than or equal to 100 micrometers, or less than or equal to 50 micrometers. Combinations of the above recited ranges are also possible (e.g., the crosslinking initiator comprises a plurality of particles having a particle size of greater than or equal to 20 micrometers and less than or equal to 300 micrometers, the crosslinking initiator comprises a plurality of particles having a particle size of greater than or equal to 50 micrometers and less than or equal to 100 micrometers).

In some embodiments, the crosslinking initiator comprises a plurality of particles having a particle density (e.g., tapped particle density) greater than or equal to 0.50 g/ml, greater than or equal to 0.60 g/ml, greater than or equal to 0.70 g/ml, greater than or equal to 0.80 g/ml, greater than or equal to 0.90 g/ml, greater than or equal to 1.00 g/ml, greater than or equal to 1.10 g/ml; greater than or equal to 1.20 g/ml, greater than or equal to 1.30 g/ml, or greater than or equal to 1.40 g/ml. In certain embodiments, the crosslinking initiator comprises a plurality of particles having a particle density (e.g., tapped particle density) less than or equal to 1.50 g/ml, less than or equal to 1.40 g/ml, less than or equal to 1.30 g/ml, less than or equal to 1.20 g/ml, less than or equal to 1.10 g/ml, less than or equal to 1.00 g/ml, less than or equal to 0.90 g/ml, less than or equal to 0.80 g/ml, less than or equal to 0.70 g/ml, or less than or equal to 0.60 g/ml. Combinations of the above recited ranges are also possible (e.g., the crosslinking initiator comprises a plurality of particles having a particle density greater than or equal to 0.50 g/ml and less than or equal to 1.50 g/ml, the crosslinking initiator comprises a plurality of particles having a particle density greater than or equal to 0.90 g/ml and less than or equal to 1.20 g/ml).

For embodiments in which the second component of the dry powder composition (e.g., second powder of a dry powder mixture) further comprises a base or basic buffer as a crosslinking initiator (e.g., sodium bicarbonate), such base or basic buffer may be present in any suitable amount. Without wishing to be bound by theory, the amount of the base or basic buffer may affect the reactivity of the dry powder composition, such as the measured time it takes for the dry powdered composition to crosslink, which is explained below in greater detail. Accordingly, in certain embodiments, it may be advantageous to select the amount of base or basic buffer in order to advance or delay hemostasis when the dry powder composition is applied to a bleeding/wound site.

The dry powder composition may comprise the base of basic buffer in any of a variety of suitable amounts. For example, in certain embodiments, the second component of the dry powder composition (e.g., dry powder mixture) comprises the basic crosslinking initiator in an amount of greater than or equal to 1 wt.%, greater than or equal to 5 wt.%, greater than or equal to 10 wt.%, greater than or equal to 15 wt.%, greater than or equal to 20 wt.%, greater than or equal to 25 wt.%, greater than or equal to 30 wt.%, greater than or equal to 35 wt.%, or greater than or equal to 40 wt.%. In certain embodiments, the second component of the dry powder composition (e.g., dry powder mixture) comprises the basic crosslinking initiator in an amount of less than or equal to 45 wt.%, less than or equal to 40 wt.%, less than or equal to 35 wt.%, less than or equal to 30 wt.%, less than or equal to 25 wt.%, less than or equal to 20 wt.%, less than or equal to 15 wt.%, less than or equal to 10 wt.%, or less than or equal to 5 wt.%. Combinations of the above recited ranges are also possible (e.g., the dry powder mixture comprises the basic crosslinking initiator in an amount of greater than or equal to 1 wt.% and less than or equal to 45 wt.%, the dry powder mixture comprises the crosslinking initiator in an amount of greater than or equal to 25 wt.% and less than or equal to 35 wt.%).

According to a specific non-limiting embodiment, the dry powder composition (e.g., dry powder mixture) comprises a first component (e.g., first dry powder) comprising PEG(SS)2, and a second component (e.g., second dry powder) comprising albumin and sodium bicarbonate, wherein the PEG(SS)2, albumin, and sodium bicarbonate are in a 1:2:1.3 mass ratio, respectively (e.g., 23 wt.% by mass PEG(SS)2, 47 wt.% by mass albumin, and 30 wt.% by mass sodium bicarbonate).

In certain embodiments, the first component (e.g., first dry powder such as PEG(SS)2) and/or the second component (e.g., second dry powder such as albumin) used herein may have a number average particle size (e.g., average cross-sectional maximum particle diameter) on the microscale. In some embodiments, the first component and/or the second component may comprise powders that have number average particle sizes (e.g., number average particle diameters) in the range of from 1 micrometer to 1000 micrometers. In some embodiments, the first component and/or the second component may comprise powders that have number average particle sizes (e.g., number average particle diameters) in the range of from 10 micrometers to 500 micrometers. The number average particle size of the first component and/or second component may be determined using spectroscopic techniques such as DLS, SEM, and/or TEM, as described above.

In any of the above described embodiments, the dry powder crosslinking hemostat composition (e.g., dry powder crosslinking hemostat mixture) may comprise other active agents or ingredients for various purposes, for example biomaterials, such as crosslinked gelatin or starch particles to allow for additional blood absorption, biologics such as thrombin to accelerate blood clotting, or any of a variety of suitable antimicrobials.

The time it takes for the dry powder composition to crosslink may determine how fast the composition forms a hemostatic hydrogel when the dry powder is applied to a bleeding/wound site. It may be beneficial for the dry powdered composition to crosslink in a substantially short time in order to quickly promote hemostasis when applied to a bleeding/wound site. In some aspects, it may be beneficial to delay formation of the hemostatic hydrogel depending on the location of the bleeding/wound site and/or the state of the patient. The "measured crosslink time" as used herein is determined by first applying the dry powder composition to a vial containing either whole blood or a solution of 0.9% normal saline as follows: to a 15.5 mm x 50 mm Fisherbrand^{™} Vial containing a 3 mm x 12.7 mm VWR^{™} brand Yellow Micro Stir Bar on a stir plate adjusted to 60 RPM, add either 631 microliters of whole blood with 33 microliters of 0.2 M CaCl₂, or 664 microliters of 0.9% normal saline, at 37 °C; to this add 166 mg of the dry powder composition (shaking lightly as need to prevent powder from sticking to the sides of the vial); the initial time (T₀) is recorded upon addition of the dry powder composition, and the timer is stopped (at T_{F}) when gelation causes the stir bar to stop spinning or when gelation occurs (as indicated by an obvious change in consistency). The stir bar may not come to a complete stop. If the stir bar continues beyond 3 minutes without an obvious change in consistency, a time of ">3 minutes" is recorded, but if the operator observes an obvious change in consistency indicating gel formation, the time of such observation is recorded and the test is discontinued even if the stir bar may not completely stop in all cases. The measured crosslink time is the time when the timer is stopped minus the initial time.

The dry powder composition may have any of a variety of suitable measured crosslink times. In some embodiments, for example, the dry powder composition may have a measured crosslink time of greater than or equal to 15 seconds, greater than or equal to 50 seconds, greater than or equal to 100 seconds, greater than or equal to 150 seconds, greater than or equal to 200 seconds, greater than or equal to 250 seconds, greater than or equal to 300 seconds, greater than or equal to 350 seconds, greater than or equal to 400 seconds, or greater than or equal to 450 seconds. In certain embodiments, the dry powder composition may have a measured crosslink time of less than or equal to 500 seconds, less than or equal to 450 seconds, less than or equal to 400 seconds, less than or equal to 350 seconds, less than or equal to 300 seconds, less than or equal to 250 seconds, less than or equal to 200 seconds, less than or equal to 150 seconds, less than or equal to 100 seconds, or less than or equal to 50 seconds. Combinations of the above recited ranges are also possible (e.g., the dry powder composition may have a measured crosslink time of greater than or equal to 15 seconds less than or equal to 600 seconds, the dry powder composition may have a measured crosslink time or greater than or equal to 15 seconds and less than or equal to 150 seconds).

In some embodiments, the measured crosslink time of the dry powder composition may depend on the type of protein and/or source of the protein. For example, when the protein comprises albumin, the measured crosslink time may depend on the source of albumin. In a certain non-limiting embodiment, for example, the protein comprises bovine serum albumin and the measured crosslink time is greater than or equal to 30 seconds and less than or equal to 50 seconds. In another non-limiting embodiment, the protein comprises human serum albumin and the measured crosslink time is greater than 40 seconds and less than 60 seconds. In yet another non-limiting embodiment, the protein may comprise recombinant human albumin and the measured crosslink time is greater than or equal to 30 seconds and less than or equal to 70 seconds.

In certain embodiments, the measured crosslink time of the dry powder composition may depend on the media that the dry powder composition is added to. For example, in some embodiments, the measured crosslink time of the dry powder composition is different when mixed with whole blood as compared to normal saline (i.e., 0.90% w/v NaCl in deionized water). In some embodiments, for example, the dry powder composition has a measured crosslink time of greater than or equal to 15 seconds, greater than or equal to 50 seconds, or greater than or equal to 100 seconds when the dry powder composition is added to whole blood. In certain embodiments, the dry powder composition has a measured crosslink time of less than or equal to 150 seconds, less than or equal to 100 seconds, or less than or equal to 50 seconds when the dry powder composition is added to whole blood. Combinations of the above recited ranges are also possible (e.g., the dry powder has a measured crosslink time of greater than or equal to 15 seconds and less than or equal to 150 seconds when the dry powder composition is added to whole blood, the dry powder has a crosslink time or greater than or equal to 50 seconds and less than or equal to 100 seconds when the dry powder is added to blood). According to certain embodiments, the dry powder composition may crosslink at a substantially faster rate in blood as compared to other media (e.g., saline), due to the presence of additional proteins (e.g., albumin) and/or cellular components in the blood.

According to certain embodiments, the dry powder composition has a measured crosslink time of greater than or equal to 15 seconds, greater than or equal to 50 seconds, greater than or equal to 100 seconds, greater than or equal to 150 seconds, or greater than or equal to 200 seconds when the composition is added to normal saline. In some embodiments, the dry powder composition has a crosslink time of less than or equal to 250 seconds, less than or equal to 200 seconds, less than or equal to 150 seconds, less than or equal to 100 seconds, or less than or equal to 50 seconds when the dry powder composition is added to normal saline. Combinations of the above recited ranges are also possible (e.g., the dry powder composition has a measured crosslink time of greater than or equal to 15 seconds and less than or equal to 250 seconds when the dry powder composition is added to normal saline, the dry powder composition has a measured crosslink time or greater than or equal to 50 seconds and less than or equal to 150 seconds when the dry powder composition is added to normal saline).

As explained above, the measured crosslink time of the dry powder composition may be affected by certain properties of the reactive powder(s) (e.g., particle size and/or particle density) of the dry powder composition. For example, in some embodiments, the protein consists essentially of particles having a particle size greater than or equal to 100 micrometers, greater than or equal to 150 micrometers, or greater than or equal to 200 micrometers, and the dry powder composition may have a measured crosslink time of greater than or equal to 15 seconds and less than or equal to 75 seconds, or in other embodiments greater than or equal to 75 seconds and less than or equal to 150 seconds, when the dry powder composition is added to whole blood. In some embodiments, the protein consists essentially of particles having a particle size of less than or equal to 250 micrometers, less than or equal to 200 micrometers, or less than or equal to 150 micrometers, and the dry powder composition has a measured crosslink time of greater than or equal to 15 seconds and less than or equal to 75 seconds when the dry powder composition is added to whole blood.

In some embodiments, the protein consists essentially of particles having a particle size of greater than or equal to 100 micrometers, greater than or equal to 150 micrometers, or greater than or equal to 200 micrometers, and the dry powder composition has a measured crosslink time of greater than or equal to 15 seconds and less than or equal to 100 seconds, or in other embodiments greater than or equal to 100 seconds and less than or equal to 150 seconds, when the dry powder composition is added to normal saline. In some embodiments, the protein consists essentially of particles having a particle size of less than or equal to 250 micrometers, less than or equal to 200 micrometers, or less than or equal to 150 micrometers, and the dry powder composition has a measured crosslink time of greater than or equal to 15 seconds and less than or equal to 75 seconds when the dry powder composition is added to normal saline.

In certain embodiments, the protein comprises a plurality of particles having a tapped particle density of greater than or equal to 0.35 g/ml, or greater than or equal to 0.40 g/ml, or greater than 0.50 g/ml, or greater than 0.60 g/ml, and the dry powder composition may have a measured crosslink time of greater than or equal to 15 seconds and less than or equal to 75 seconds, or in other embodiments greater than or equal to 75 seconds and less than or equal to 150 seconds, when the dry powder composition is added to whole blood. In some embodiments, the protein comprises a plurality of particles having a tapped particle density of less than or equal to 0.45 g/ml or less than or equal to 0.40 g/ml, and the composition may have a measured crosslink time of greater than or equal to 15 seconds and less than or equal to 75 seconds, or in other embodiments greater than or equal to 75 seconds and less than or equal to 150 seconds, when the dry powder composition is added to whole blood.

In some embodiments, the protein comprises a plurality of particles having a tapped particle density of greater than or equal to 0.35 g/ml, or greater than or equal to 0.40 g/ml, or greater than 0.50 g/ml, or greater than 0.60 g/ml, and the dry powder composition may have a measured crosslink time of greater than or equal to 15 seconds and less than or equal to 100 seconds, or in other embodiments greater than or equal to 100 seconds and less than or equal to 150 seconds when the dry powder composition is added to normal saline. In some embodiments, the protein comprises a plurality of particles having a tapped particle density of less than or equal to 0.45 g/ml or less than or equal to 0.40 g/ml, and the dry powder composition may have a measured crosslink time of greater than or equal to 15 seconds and less than or equal to 100 seconds, or in other embodiments greater than or equal to 100 seconds and less than or equal to 150 seconds, when the dry powder composition is added to normal saline.

In certain cases, the measured crosslink time of the dry powder composition may be affected by the relative amount of base or basic buffer. For example, in certain embodiments, the dry powder composition may comprise a crosslinking initiator (e.g., a base or basic buffer), and the amount of the crosslinking initiator may affect the time it takes for the composition to crosslink in various media (e.g., a solution of blood, a solution of saline) due to changes in the pH value of the solution. In some embodiments, the dry powder comprises greater than or equal to 1 wt.%, greater than or equal to 5 wt.%, greater than or equal to 10 wt.%, greater than or equal to 15 wt.%, greater than or equal to 20 wt.%, greater than or equal to 25 wt.%, greater than or equal to 30 wt.%, greater than or equal to 35 wt.%, or greater than or equal to 40 wt.% by mass base or basic buffer, and the composition has a measured crosslink time of greater than or equal to 15 seconds and less than or equal to 150 seconds when the dry powder composition is added to whole blood. In some embodiments, the dry powder comprises less than or equal to 45 wt.%, less than or equal to 40 wt.%, less than or equal to 35 wt.%, less than or equal to 30 wt.%, less than or equal to 25 wt.%, less than or equal to 20 wt.%, less than or equal to 15 wt.%, less than or equal to 10 wt.%, or less than or equal to 1 wt.% by mass base or basic buffer, and the dry powder composition has a measured crosslink time of greater than or equal to 15 seconds and less than or equal to 150 seconds when the dry powder composition is added to whole blood.

In some embodiments, the dry powder composition comprises greater than or equal to 1 wt.%, greater than or equal to 5 wt.%, greater than or equal to 10 wt.%, greater than or equal to 15 wt.%, greater than or equal to 20 wt.%, greater than or equal to 25 wt.%, greater than or equal to 30 wt.%, greater than or equal to 35 wt.%, or greater than or equal to 40 wt.% by mass base or basic buffer, and the dry powder composition has a measured crosslink time of greater than or equal to 15 seconds and less than or equal to 250 seconds, or in other embodiments greater than or equal to 250 seconds and less than or equal to 400 seconds, when the dry powder composition is added to normal saline. In some embodiments, the dry powder composition comprises less than or equal to 45 wt.%, less than or equal to 40 wt.%, less than or equal to 35 wt.%, less than or equal to 30 wt.%, less than or equal to 25 wt.%, less than or equal to 20 wt.%, less than or equal to 15 wt.%, less than or equal to 10 wt.%, or less than or equal to 1 wt.% by mass base or basic buffer, and the dry powder composition has a measured crosslink time of greater than or equal to 15 seconds and less than or equal to 250 seconds when the dry powder composition is added to normal saline.

The composition as disclosed and presently claimed can be used in methods for controlling bleeding. For example, in some embodiments, the method comprises applying any of the above described crosslinkable dry powder components to a bleeding/wound site (e.g., bleeding tissue). In certain embodiments, upon exposure to aqueous liquid in the bleeding/wound site, the crosslinkable dry powder crosslinks to form a hemostatic hydrogel capable of stopping and/or reducing bleeding at the bleeding/wound site.

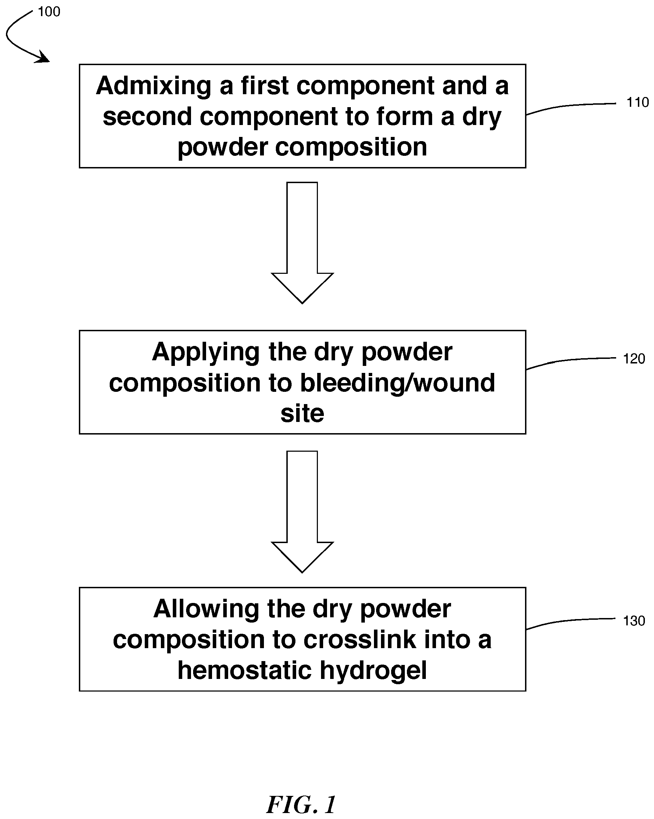

FIG. 1, for example, shows steps in an exemplary method for forming a hemostatic hydrogel with a dry powdered mixture. In method 100, step 110 comprises admixing a first component (e.g., first dry powder) and the second component (e.g., second dry powder) to form a dry powder composition (e.g., dry powder mixture). Step 120 comprises applying the dry powder composition (e.g., dry powder mixture) to a bleeding/wound site, and step 130 comprises allowing the dry powder composition (e.g., dry powder mixture) to crosslink into a hemostatic hydrogel upon exposure to aqueous liquid in the bleeding/wound site, wherein the hemostatic hydrogel is capable of stopping and/or reducing bleeding at the bleeding/wound site. Alternatively (not pictured), in some embodiments, the first and second powder components could be applied separately to the site simultaneously or sequentially without prior formation of a powder mixture.

The hemostatic hydrogel (e.g., resulting from application of the dry powder composition to a bleeding/wound site) may be characterized by one or more measured viscoelastic properties, in some embodiments, using an ElastoSens^{™} Bio² instrument from Rheolution, Inc. (Montreal, Quebec, Canada). In certain embodiments, for example, the ElastoSens^{™} Bio² instrument may be used to measure, for example, the shear elastic modulus (G'), the gelation rate (*d*G'/*d*t), and/or other relevant viscoelastic properties. In some embodiments, the shear elastic modulus (G') may be measured as a function of time as the dry powder composition hydrates with a fluid and polymerizes. It may be beneficial, in some embodiments, for the hemostatic hydrogel to have a sufficiently large shear elastic modulus to prevent or reduce elastic deformation of the hemostatic hydrogel after application to a bleeding/wound site. In certain embodiments, it may be beneficial for the hemostatic hydrogel to have a sufficiently fast gelation rate in order to quickly promote hemostasis when the dry powder composition is applied to a bleeding/wound site.

The ElastoSens^{™} Bio² instrument may be operated according to the following procedure. The ElastoSens^{™} Bio² instrument is first calibrated (e.g. each day of use) according to a standard calibration procedures using the provided plastic calibration inserts and the associated instrument software (ElastoView^{™}, version 18.12). After calibration, the sample holders are then placed in an incubator at 37 °C for 20 minutes. The sample holders are placed into the thermal chamber of the instrument and secured such that the sample holders cannot move. A new test is initiated using the associated instrument software. Next, 0.5 g of the dry powder composition is weighed and poured into the sample holder. A single pipette or multi-channel pipette is then filled with the hydration fluid at 37 °C (e.g. normal saline or whole blood), which is then released into the sample holder in a circular motion to ensure that all powder is evenly covered with the hydration fluid. Once sample loading is complete, the lid is of the instrument is closed and the test is started immediately.

The hemostatic hydrogel may have any of a variety of shear elastic moduli. According to certain embodiments, for example, the hemostatic hydrogel may have a maximum shear elastic modulus (G') greater than or equal to 1000 Pa, greater than or equal to 2000 Pa, greater than or equal to 3000 Pa, greater than or equal to 4000 Pa, greater than or equal to 5000 Pa, greater than or equal to 6000 Pa, greater than or equal to 7000 Pa, greater than or equal to 8000 Pa, greater than or equal to 9000 Pa, greater than or equal to 10000 Pa, greater than or equal to 11000 Pa, greater than or equal to 12000 Pa, greater than or equal to 13000 Pa, greater than or equal to 14000 Pa, greater than or equal to 15000 Pa, greater than or equal to 16000, greater than or equal to 18,000, or greater than or equal to 19000 Pa. In certain embodiments, the hemostatic hydrogel has a shear elastic modulus less than or equal to 20000 Pa, less than or equal to 19000 Pa, less than or equal to 18000 Pa, less than or equal to 17000 Pa, less than or equal to 16000 Pa, less than or equal to 15000 Pa, less than or equal to 14000 Pa, less than or equal to 13000 PA, less than or equal to 12000 Pa, less than or equal to 11000 Pa, less than or equal to 10000 Pa, less than or equal to 9000 Pa, less than or equal to 8000 Pa, less than or equal to 7000 Pa, less than or equal to 6000 Pa, less than or equal to 5000 Pa, less than or equal to 4000 Pa, less than or equal to 3000 Pa or less than or equal to 2000 Pa. Combinations of the above recited ranges are also possible (e.g., the hemostatic hydrogel has a shear elastic modulus of greater than or equal to 1000 Pa and less than or equal to 20000 Pa, the hemostatic hydrogel has a shear elastic modulus of greater than or equal to 4000 Pa and less than or equal to 10000 Pa). Other ranges are also possible.

The dry powder composition may have any of a variety of suitable gelation rates. As used herein, the term "gelation rate" refers to the speed at which hydrogel formation occurs over time, measured as the derivative of the shear elastic modulus over the derivative of time (*d*G'/*d*t)*.* In some embodiments, for example, the dry powder composition has a gelation rate of greater than or equal to 20 Pa/sec, greater than or equal to 50 Pa/sec, greater than or equal to 100 Pa/sec, greater than or equal to 150 Pa/sec, greater than or equal to 200 Pa/sec, greater than or equal to 250 Pa/sec, greater than or equal to 300 Pa/sec, greater than or equal to 350 Pa/sec, greater than or equal to 400 Pa/sec, or greater than or equal to 450 Pa/sec. In certain embodiments, the dry powder composition has a gelation rate less than or equal to 500 Pa/sec, less than or equal to 450 Pa/sec, less than or equal to 400 Pa/sec, less than or equal to 350 Pa/sec, less than or equal to 300 Pa/sec, less than or equal to 250 Pa/sec, less than or equal to 200 Pa/sec, less than or equal to 150 Pa/sec, less than or equal to 100 Pa/sec, or less than or equal to 50 Pa/sec. Combinations of the above recited ranges are also possible (e.g., the dry powder composition has a gelation rate of greater than or equal to 20 Pa/sec and less than or equal to 500 Pa/sec, the dry powder composition has a gelation rate of greater than or equal to 50 Pa/sec and less than or equal to 250 Pa/sec). Other ranges are also possible.

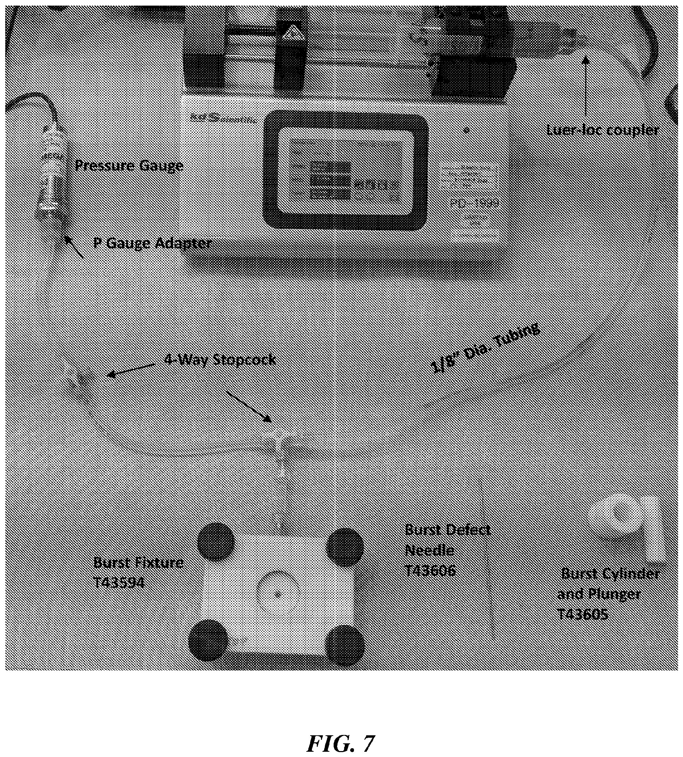

According to certain embodiments, the adherence of the hemostatic hydrogel compositions described herein once formed upon crosslinking can be determined by a burst pressure model based on ASTM F2392-04, (the Standard Test Method for Surgical Sealants). According to certain embodiments, the test is designed to determine the pressure needed to rupture a sealant patch covering a simulated liquid leak and indirectly measure the adhesion property of the sealant to simulated tissue. Briefly, a pressure gauge, syringe pump, and burst fixture are assembled as shown in FIG. 7. The burst fixture is described in more detail in the ASTM F2392-04 standard protocol. All tubing is filled with 0.9% saline that has been dyed with a colored food dye (2-3 drops per L) so that no air bubbles are present in the tubing. Once the tubing is filled with the 0.9% saline, the line to all tubing is opened and the syringe is pushed until the saline starts to come out of the top hole of the burst fixture. Next, an appropriately sized piece of collagen is cut and then rinsed a minimum of three times in deionized water in a 500 mL beaker to remove glycerol. The collagen is cut into 2 inch wide strips and transferred to a new 500 mL beaker of deionized water to soak for a minimum of ten minutes. Next, the top of the burst fixture is removed and a single piece of collagen is placed over the opening of the burst fixture. The top of the burst fixture is then placed over the collagen and secured tightly. A burst defect needle (e.g., as shown in FIG. 7) is used to create a single defect in the center of the collagen by piercing the needle straight down the center of the hole in the burst fixture and straight back up. The burst cylinder (e.g., as shown in FIG. 7) is then placed on top of the fixture on top of the collagen. The dry powder composition is weighed in amount of 166 mg and poured into the burst cylinder. Next, 250 microliters of saline is pipetted into the cylinder directly onto the dry powder composition. Immediately after pipetting the saline, the burst plunger (e.g., as shown in FIG. 7) is placed into the burst cylinder on top of the hydrated composition. The hydrated composition is allowed to polymerize for 2.5 minutes. Next, the burst cylinder is removed by holding down the top of the burst plunger and pulling the cylinder straight up and off the top surface of the burst fixture. Once the cylinder has been released from the burst fixture, the plunger and cylinder are tilted and lifted to the side and then up and away from the polymerized composition. The sample is then inspected to ensure that removal of the cylinder and/or plunger did not disrupt the polymerized composition. A computer and appropriate software (e.g., Omega Digital Transducer Application, v. 2.3.0.300) is then used to record pressure readings. The polymerized composition is observed until the sample and/or substrate fails, a large pressure drop occurs, or the pressure plateaus for 30 seconds. Substrate failure occurs if the collagen rips separate from the material. Cohesive failure occurs if there is a defect through the polymerized composition. Cohesive and substrate failure occurs if the collagen and polymerized composition rip away from the defect site. Adhesive failure occurs if there is a defect between the material and the substrate interface.

In certain embodiments, the burst pressure of the hemostatic hydrogel measured by such test is greater than or equal to 10 mm Hg, greater than or equal to 50 mm Hg, greater than or equal to 100 mm Hg, greater than or equal to 150 mm Hg, greater than or equal to 200 mm Hg, greater than or equal to 250 mm Hg, greater than or equal to 300 mm Hg, or greater than or equal to 350 mm Hg. In certain embodiments, the burst pressure of the hemostatic hydrogel is less than or equal to 400 mm Hg, less than or equal to 350 mm Hg, less than or equal to 300 mm Hg, less than or equal to 250 mm Hg, less than or equal to 200 mm Hg, less than or equal to 150 mm Hg, less than or equal to 100 mm Hg, or less than or equal to 50 mm Hg. Combinations of the above recited ranges are also possible (e.g., the burst pressure of the hemostatic hydrogel is greater than or equal to 10 mm Hg and less than or equal to 350 mm Hg).

According to certain embodiments, the hemostatic efficiency of the hemostatic hydrogel compositions can be determined in animal models of controlled bleeding by the number of cycles of manually applied pressure required to achieve hemostasis upon application of the dry powder mixture hemostats, as described above. In some embodiments, the number of pressure cycles required to achieve hemostasis upon application of the dry powder mixture hemostats is greater than or equal to 1 cycles, greater than or equal to 2 cycles, or greater than or equal to 3 cycles. According to certain embodiments, the number of pressure cycles required to achieve hemostasis upon application of the dry powder mixture hemostats is less than or equal to 4 cycles, less than or equal to 3 cycles, or less than or equal to 2 cycles. Combinations of these ranges are also possible (e.g., the number of pressure cycles required to achieve hemostasis upon application of the dry powder mixture hemostats is greater than or equal to 1 cycle and less than or equal to 3 cycles).

According to certain embodiments, the hemostatic efficiency of the hemostatic hydrogel compositions can also be determined by the time it takes to achieve hemostasis upon application of the dry powder mixture hemostats described herein. According to certain embodiments, the time it takes to achieve hemostasis upon application of the dry powder mixture hemostats is less than or equal to 2.5 minutes, less than or equal to 2.0 minutes, less than or equal to 1.5 minutes, less than or equal to 1.0 minute, less than or equal to 0.5 minutes, or less than or equal to 0.2 minutes.

In some embodiments, the hemostatic efficiency of the hemostatic hydrogel compositions can also be determined by the percent of treated defects achieving and maintaining hemostasis upon application of the dry powder mixture hemostats described herein. According to some embodiments, the percent of treated defects achieving and maintaining hemostasis upon application of the dry powder mixture is greater than or equal 70%, greater than or equal to 75%, greater than or equal to 80%, greater than or equal to 85%, greater than or equal to 90%, greater than or equal to 95%, or greater than or equal to 99%, or 100%.

In certain embodiments, the dry powder composition (e.g., dry powder mixture) may be prepared and/or admixed by any of a variety of suitable methods. For example, in some embodiments, the dry powder mixture may be prepared by ball milling (e.g., the dry powder mixture may be ground in a ball mill). In certain embodiments, the dry powder mixture may be prepared and/or admixed by grinding with a mortar and pestle.

According to certain embodiments, the dry powder composition (e.g., dry powder mixture) can be provided (e.g., packaged) as a sealed, admixed powder. For example, in some cases, the dry powder mixture is provided in a vial and/or ampoule (e.g., a flame-sealed vial and/or ampoule). In certain embodiments, the vial and/or ampoule containing the dry powder mixture may be attached to a sprayer that is capable of spraying the powder (e.g., onto a bleeding/wound site). In yet other embodiments, the dry powdered components could be formed into one or two component paste, putty or wax forms for use as crosslinking patching/filling materials - e.g. in bone bleeding applications.

The dry, powdered hemostatic compositions described herein may be provided in any of a variety of suitable forms. In some embodiments, dry, powdered hemostatic compositions comprise at least a first dry powder and a second dry powder (and optionally a third dry powder, etc.). The first dry powder may comprise a first component described above. For example, in some embodiments, the first dry powder comprises a difunctionalized polyalkylene oxide-based polymer comprising electrophilic groups (e.g., PEG(SS)2). The second powder may comprise a second component described above. For example, the second dry powder may comprise a protein (e.g., albumin).

In some embodiments, the dry, powdered hemostatic composition comprises at least one composite powder. For example, the dry, powdered hemostatic composition may comprise a single dry powder that is a composite of some or all of the components described above (e.g., a single dry powder of particles comprising a nucleophilic component (e.g., albumin) coated on an electrophilic component (e.g., PEG(SS)2). Such a powder of coated particles could be prepared, for example, by spray-coating using a solvent in which the components are unreactive (e.g., a non-aqueous solvent). In some embodiments, the dry, powdered composition comprises a dry powder mixture of multiple dry powders (e.g., the first dry powder and second dry powder). In other embodiments, the first dry powder and the second dry powder are provided as separated, unmixed powders (e.g., as packaged), and are combined prior to or upon use (e.g., prior to or as applying to a bleeding/wound site).

Subject to the appended claims, the crosslinking initiator may be incorporated in any of a variety of suitable forms. For example, in some embodiments, the first dry powder comprises the crosslinking initiator. In some such embodiments, the composition comprises a first dry powder comprising a first component comprising a powder mixture of or composite particles of a reactive electrophilic compound (e.g., a difunctionalized polyalkylene oxide-based polymer functionalized with electrophilic groups) and the crosslinking initiator (e.g., a base or basic buffer). In some embodiments, a first dry powder comprises particles of the reactive electrophilic compound spray-coated with crosslinking initiator, or vice versa. In some embodiments, the second dry powder comprises the crosslinking initiator. In some such embodiments, the composition comprises a second dry powder comprising a second component comprising a power mixture of or composite particles of a reactive nucleophilic compound (e.g., a protein such as albumin) and the crosslinking initiator (e.g., a base or basic buffer). In some embodiments, a second dry powder comprises particles of the reactive nucleophilic compound spray-coated with crosslinking initiator, or vice versa.

In some embodiments, the crosslinking initiator is provided as a powder separate from the reactive electrophilic (e.g., PEG(SS)2) or nucleophilic (e.g., albumin) compounds above. For example, in some embodiments, the dry, powdered hemostatic composition comprises a first dry powder comprising the first reactive electrophilic component, a second dry powder comprising a reactive nucleophilic compound (e.g., a protein such as albumin), and a third dry powder comprising the crosslinking initiator (e.g., a base or basic buffer). The first, second, and third dry powders may be packaged separately or combined as a dry powder mixture.

In some embodiments, the dry, powdered hemostatic composition comprises a single dry powder comprising composite particles formed of a reactive nucleophilic compound, a crosslinking initiator, and a reactive electrophilic compound. For example, the dry, powdered hemostatic composition comprises a single dry powder of particles formed of a nucleophilic component (e.g., albumin) coated with a crosslinking initiator (e.g., a base or basic buffer), which is in turn coated with an electrophilic component (e.g., PEG(SS)2). Other configurations of the components are also possible. Such a powder of coated particles could be prepared, for example, by spray-coating using a solvent in which the components are unreactive (e.g., a non-aqueous solvent).

In certain embodiments, it may be advantageous to reduce the physical contact between certain components of the composition prior to applying the dry powder mixture to a bleeding/wound site. In some embodiments, for example, physical contact between the difunctionalized polymeric composition (e.g., PEG(SS)2) and one or more components of the dry powdered composition, such as the crosslinking initiator (e.g., base or basic buffer) or the protein (e.g. albumin), may be reduced prior to applying the dry powder mixture to a bleeding/wound site. In some embodiments, for example, reducing the physical contact between the difunctionalized polymeric composition and the crosslinking initiator may avoid chemical reactions between the two that can occur during storage, thereby increasing the overall shelf-life of the dry powder composition. As would be understood by a person of ordinary skill in the art, the difunctionalized polymeric composition (e.g., PEG(SS)2), in some embodiments, is temperature and/or moisture sensitive. For example, in certain non-limiting embodiments, one or more ester bonds of the difunctionalized polymeric composition may be hydrolyzed in the presence of moisture (e.g., inherent in the atmosphere), which, in some embodiments, is facilitated and/or accelerated by the presence of the crosslinking initiator (e.g., base or basic buffer). Therefore, in certain embodiments, reducing the physical contact between the difunctionalized polymeric composition and the crosslinking initiator may inhibit such hydrolysis from occurring during product storage, therefore increasing the overall shelf-life of the dry powder composition. It may be advantageous, in some embodiments, to reduce the physical contact between the difunctionalized polymeric composition and the protein (e.g., albumin) in order to prevent hydrolysis that may occur when the difunctionalized polymeric composition is in contact with inherent moisture present within the protein.

In certain embodiments, reducing the physical contact comprises lowering (or eliminating) the surface area (e.g., points of contact) between the difunctionalized polymeric composition and one or more components of the dry powdered composition, such as the crosslinking initiator (e.g., base or basic buffer) or the protein (e.g., albumin). In some embodiments, for example, the protein and/or crosslinking initiator may be manipulated such that there is limited physical contact between the difunctionalized polymeric composition and the crosslinking initiator. According to some embodiments, for example, the protein may be roller compacted and/or granulated with the crosslinking initiator (e.g., base or basic buffer) prior to mixing with the difunctionalized polymeric composition. In other embodiments, the protein may be spray coated onto and/or over the crosslinking initiator (e.g., base or basic buffer) prior to mixing with the difunctionalized polymeric composition, such that, upon mixing, the difunctionalized polymer composition is substantially only in contact with the protein and not the crosslinking initiator during storage.

In certain embodiments, the difunctionalized polymeric composition may be manipulated such that there is limited physical contact between the difunctionalized polymeric composition and one or more other components of the dry powdered composition (e.g., the crosslinking initiator and/or the protein). In some embodiments, for example, the difunctionalized polymeric composition may be coated with an inert material. The inert material may be, in some embodiments, a polymer. Any of a variety of suitable polymers that are suitable to coat and will not destroy or substantially degrade the reactivity of the difunctionalized polymeric composition may be employed. Suitable polymers include those polymers that are biodegradable, biocompatible, and/or soluble or water dispersible. In some embodiments, for example, such polymer is polyvinylpyrrolidone (PVP) or hydroxypropyl methylcellulose (HPMC).

In certain cases, a hemostatic article comprises a powdered hemostatic composition used with, in contact with, or otherwise associated with a tamponade device. Applying a reactive, dry, powdered hemostatic composition to a bleeding wound using a tamponade may further reduce or stop bleeding at a bleeding/wound site upon formation of a hydrogel due to cross-linking of components of the hemostatic composition compared to use of the dry, powdered hemostatic compositions alone. The presence of a tamponade can, in some cases, improve the efficacy of dry, powdered hemostatic compositions in applications where high flow bleeding occurs. In some embodiments, the tamponade is a biodegradable tamponade. Combining a reactive, dry, powdered hemostatic composition with a biodegradable tamponade can, in some cases, provide for a hemostatic device that can be easily applied to a wound or bleeding site while improving certain performance aspects of the reactive hemostats. For example, applying a dry, powdered hemostatic composition in contact with a biodegradable tamponade can, in some cases, mitigate adhesion between, polymerized hemostatic composition and, for example, a non-biodegradable applicator or material otherwise used to contact the hemostatic composition, such as gauze.

In some cases, the reactive, dry, powdered hemostatic composition that is used with (e.g. is in contact with) the tamponade is one of the dry, powdered hemostatic compositions described above. For example, in some embodiments, a dry, powdered hemostatic composition comprising a first component comprising a difunctionalized polymer (e.g., PEG(SS)2) and a second component comprising a protein (e.g., albumin) is in contact with the tamponade.

The tamponade can comprise any of a variety of suitable materials. In certain cases, the tamponade is in the form of a foam having any suitable form factor or aspect ratio. For example, the tamponade may be in the form of a sheet or layer. In some cases, the tamponade is or comprises collagen (e.g., collagen foam). One such collagen-containing tamponade is an Ultrafoam^{™} tamponade. In other cases, the tamponade is or comprises gelatin (e.g., a gelatin foam). One such gelatin-containing tamponade is Gelfoam^{™}. In certain embodiments, the tamponade comprises carboxymethylcellulose (CMC). In some embodiments, the tamponade comprises a polysaccharide. As an example, in some embodiments, the tamponade comprises a starch foam. In certain embodiments, the starch foam may be degradable, dispersible, and/or soluble. The reactive, dry, powdered hemostatic composition in contact with the tamponade (e.g., the Ultrafoam^{™} tamponade, the tamponade comprising a starch foam, etc.) may be located relatively close to the surface of the tamponade. For example, in some cases the reactive, dry, powdered hemostatic composition is in contact predominately with an external surface of the tamponade. In certain embodiments, the reactive, dry, powdered hemostatic composition is contained within the tamponade (e.g., the tamponade is impregnated with the reactive, dry, powdered hemostatic composition).

In some embodiments, the reactive, dry, powdered hemostatic composition is applied to a bleeding/wound site at a different time than is the tamponade. For example, in some embodiments, a dry, powdered hemostatic composition is applied to a bleeding/wound site, and subsequently the tamponade is applied to the bleeding/wound site (optionally with an application of steady or intermittent manual pressure to the tamponade). However, in certain cases, the reactive, dry, powdered hemostatic composition and the tamponade (e.g., containing the dry, powdered hemostatic composition) are applied to the bleeding/site at the same time.

### EXAMPLE 1

The following example describes the hemostatic efficacy of a PEG(SS)2-based dry powder mixture in a porcine spleen biopsy defect model. The dry powder mixture was prepared by mixing PEG(SS)2 (119 mg), bovine serum albumin (BSA) (228 mg) and 153 mg of sodium bicarbonate (153 mg) in a mass ratio of 1:2:1.3. The material was tested for hemostatic efficacy in a porcine spleen biopsy defect bleeding model. Briefly, a 10 mm diameter biopsy defect to a depth of about 5mm was made in the spleen. Next, 0.5 g of the dry powder mixture was applied to the defect and a 3 cm x 3 cm piece of Ultrafoam was placed on top of the powder to prevent it from sticking to the gauze used for application of pressure. Cessation of bleeding was assessed after thirty second tamponade or pressure cycles. The dry powder mixture had improved efficacy compared to other commercially available hemostatic agents (see Table 1). The material worked consistently (9 out of 10 in one pressure cycle) and adhered tenaciously to the tissue. Table 1. Hemostatic efficacy of PEG(SS)2-based dry powder mixture in a porcine spleen biopsy defect model.

| **Sample** | **Description** | **# of Defects Treated** | **# of Pressure Cycles Required to Achieve Hemostasis (mean ± std. dev.)** | **Time to Hemostasis (min) (mean ± std. dev.)** | **% of Defects Achieving and Maintaining Hemostasis (2 min. Observation)** |
|---|---|---|---|---|---|
| PEG(SS)2-based dry powder mixture + Ultrafoam^{™} | powder + foam sponge | 10 | 1.2 ± 0.6 | 1.1 ± 0.4 | 100 |
| Ultrafoam^{™} | foam sponge | 8 | 2.4 ± 1.3 | 2.2 ± 1.1 | 75 |
| Floseal^{™} | flowable paste (gelatin + thrombin) | 7 | 1.9 ± 1.1 | 1.7 ± 0.9 | 71 |

### EXAMPLE 2

The following example describes the hemostatic efficacy of the PEG(SS)2-based dry powder mixture prepared in Example 1 in a heparinized porcine spleen biopsy defect model. Heparin was used to mimic clinically relevant coagulopathies and heparinization during cardiovascular surgery. Briefly, an initial IV bolus of 150 U heparin/kg was given to increase the activated clotting time (ACT) to about 2-3× baseline. Testing of the dry powder mixture followed the description in Example 1 and the results were compared to other commercially available hemostatic agents (see Table 2). The dry powder mixture worked consistently in one pressure cycle and adhered tenaciously to the tissue. Efficacy of the dry powder mixture in the heparinized model was similar to the efficacy in the non-heparinized model described in Example 1. In contrast, the competitive products had reduced efficacy in the heparinized model.

**Table 2. Hemostatic efficacy of PEG(SS)2-based dry powder mixture in a heparinized porcine spleen biopsy defect model.**

| **Sample** | **Description** | **# of Defects Treated** | **# of Pressure Cycles Required to Achieve Hemostasis (mean ± std. dev.)** | **Time to Hemostasis (min) (mean ± std. dev.)** | **% of Defects Achieving and Maintaining Hemostasis (2 min. Observation)** |
|---|---|---|---|---|---|
| PEG(SS)2-based dry powder mixture + Ultrafoam^{™} | powder + foam sponge | 6 | 1.0 ± 0.0 | 1.0 ± 0.0 | 100 |
| Ultrafoam^{™} | foam sponge | 6 | 7.0 ± 3.5 | 5.6 ± 3.1 | 17 |
| Floseal^{™} | flowable paste (gelatin + thrombin) | 6 | 4.8 ± 3.4 | 4.2 ± 2.7 | 0 |

### EXAMPLE 3

The following example describes the hemostatic efficacy of the PEG(SS)2-based dry powder mixture prepared in Example 1 in a heparinized porcine spleen abrasion model. The abrasion was made using the rough surface of an electrocautery pad over an area of 0.5" x 0.5" and created an oozing bleed. Heparin was used to mimic clinically relevant coagulopathies and heparinization during cardiovascular surgery. Briefly, an initial IV bolus of 150 U heparin/kg was given to increase the activated clotting time (ACT) to about 2-3× baseline. Five hundred milligrams of the dry powder mixture was sprinkled over the bleeding area, no tamponade or pressure cycle was applied, and hemostasis was assessed after an initial 30 seconds. If hemostasis was achieved a two minute observation period was used to assess rebleeds. If hemostasis was achieved and maintained, the wound site was irrigated with saline and re-assessed for hemostasis. Results were compared to other commercially available hemostatic agents (see Table 3). The dry powder mixture worked better than the competitive products and adhered tenaciously to the tissue.

**Table 3. Hemostatic efficacy of PEG(SS)2-based dry powder mixture in a heparinized porcine spleen abrasion model.**

| **Sample** | **Description** | **# of Defects Treated** | **% Hemostasis Achieved** | **% Hemostasis Maintained** | **% Hemostasis Maintained After Irrigation** |
|---|---|---|---|---|---|
| PEG(SS)2 dry powder mixture | powder | 8 | 75 | 75 | 75 |
| Raplixa^{™} Powder | powder (trehalose / fibrinogen / thrombin) | 8 | 50 | 13 | 0 |
| Surgicel^{™} Powder | oxidized regenerated cellulose powder | 5 | 0 | 0 | 0 |

### EXAMPLE 4

The following example describes the demonstration of sealant properties of the PEG(SS)2-based dry powder mixture prepared in Example 1 in an *in vitro* burst pressure model (based on ASTM F2392-04; Standard Test Method for Surgical Sealants). The test is designed to determine the pressure needed to rupture a sealant patch covering a simulated liquid leak and indirectly measure the adhesion property of the sealant to simulated tissue. Briefly, a hydrated collagen casing membrane was secured in a burst pressure fixture and a hole was created with a 3-0 RB 1 suture needle. The dry powder mixture was applied to the membrane and hydrated with saline. A syringe pump supplied saline to the fixture at a flow rate of 2 ml/min and burst pressure at failure was recorded. The material tested at two conditions of amount and cure time exhibited sealant properties (see Table 4).

**Table 4. Sealant properties of PEG(SS)2-based dry powder mixture in a burst pressure model.**

| **Test** | **Amount of Powder Mixture (mg)** | **Amount of Saline Hydration (µl)** | **Cure Time Allowed Prior to Test** | **Burst Pressure (mm Hg)** |
|---|---|---|---|---|
| Minimal dry powder mixture / short cure time (n = 13) | 35 | 40 | 15 sec | 90 ± 58 |
| Moderate dry powder mixture / long cure time (n = 6) | 166 | 250 | 5 min | 236 ± 78 |

### EXAMPLE 5

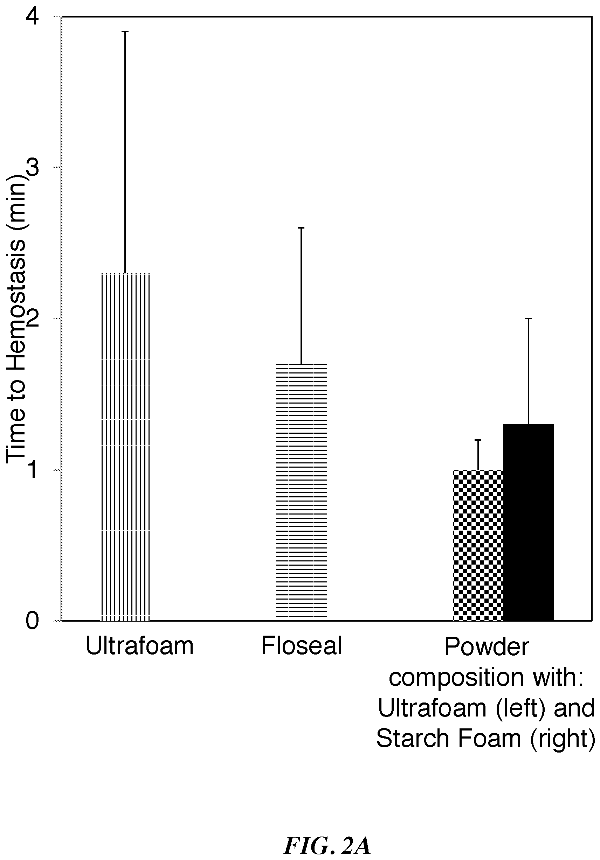

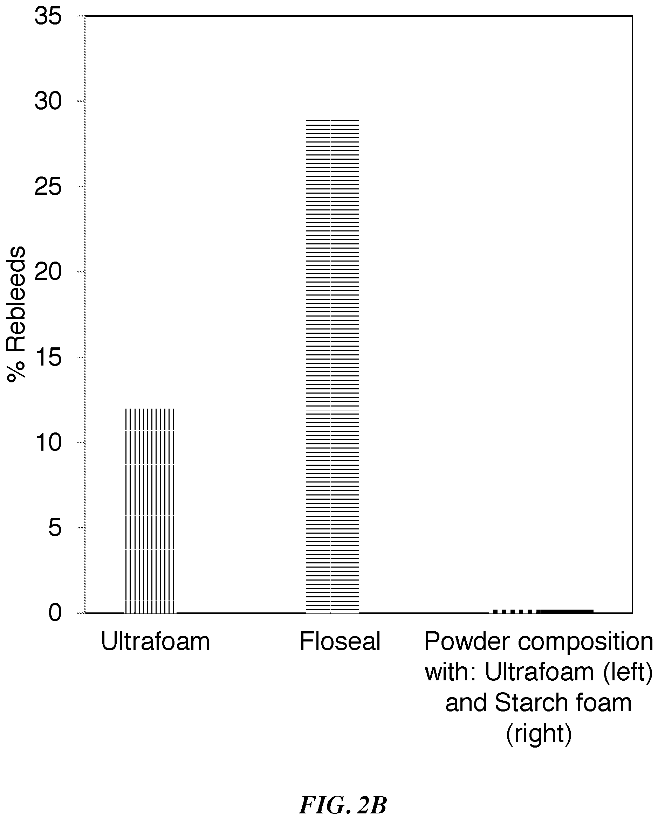

The following examples describes the hemostatic efficacy of a PEG(SS)2 dry powder composition in a porcine spleen biopsy defect bleeding model (modified wet field model). The material prepared in Example 1 was tested for hemostatic efficacy in a porcine spleen biopsy defect bleeding model as described previously in Example 1. However, in order to increase the hemostatic challenge in a realistic surgical scenario, the material was applied to an actively bleeding site, instead of blotting the defect dry prior to placement. Briefly, a 10 mm diameter biopsy defect to a depth of ~5 mm was made in the spleen. The defect was allowed to fill with blood and then 0.5 g of the reactive powder mixture was applied to the defect and a 3 cm x 3 cm piece of Ultrafoam^{™} or a tamponade comprising a starch foam was placed on top of the powder to prevent it from sticking to the gauze used for application of tamponade. Cessation of bleeding was assessed after 30 second tamponade cycles. The dry reactive powder mixture had improved efficacy compared to other commercially available hemostatic agents, as shown by the reduced time to achieve hemostasis in FIG. 2A and the fewer percent rebleeds during a 2 minute observation after hemostasis in FIG. 2B.

### EXAMPLE 6

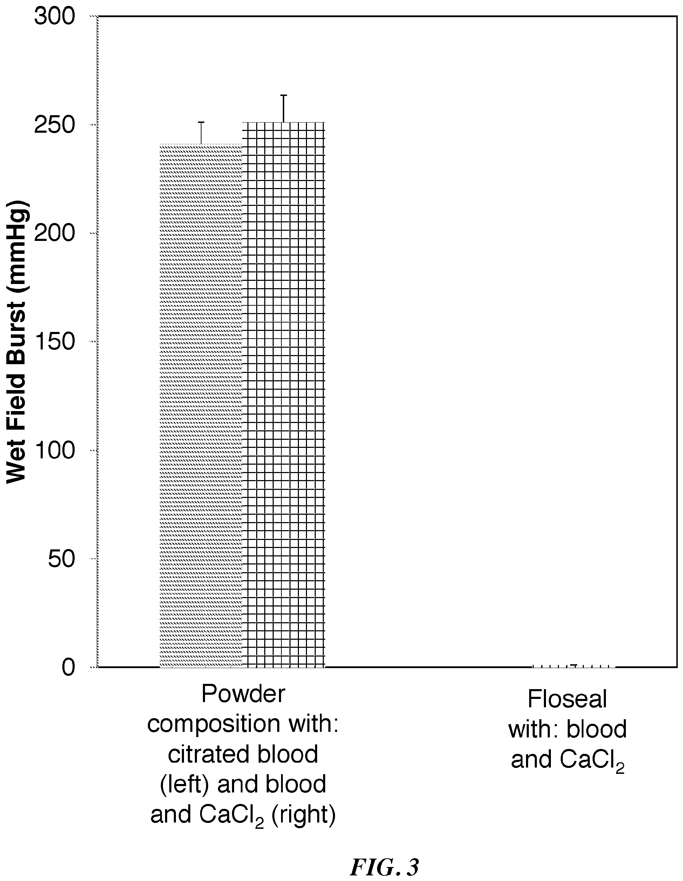

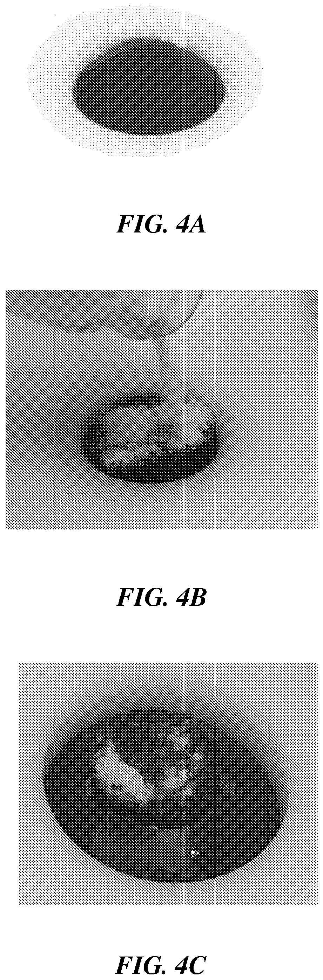

The following example describes the evaluation of sealant properties of a PEG(SS)2 dry powder composition in an in vitro burst pressure model (e.g., a modified wet field model). The material prepared in Example 1 was tested in an in vitro burst pressure model (based on ASTM F2392-04; Standard Test Method for Surgical Sealants) as described previously in Example 4. However, in order to increase the adherence challenge, the material was applied with a layer of blood already on the collagen membrane instead of blotting the membrane dry prior to placement. Citrated whole sheep's blood (0.3 ml) was applied to the surface of the collagen membrane (see FIG. 4A). This amount had a depth of approximately 1.5 mm. The material prepared in Example 1 was applied to the layer of blood and allowed to cure for 5 minutes (see FIG. 4B and FIG. 4C). A syringe pump supplied saline to the fixture at a flow rate of 2 ml/min and burst pressure at failure was measured. The material exhibited sealant properties through the layer of blood, as shown in FIG. 3, and performed orders of magnitude better than a commercially available hemostatic agent.

### EXAMPLE 7

The following example describes the measured crosslink time of a PEG(SS)2 dry powder composition. The crosslinking rate of the material prepared in Example 1 was tested in a measured crosslink time assay as described above. Briefly, 664 microliters of saline was added to a 15.5 mm x 50 mm vial and stirred with a 3 mm x 12.7 mm micro stir bar at 60 rpm. Then, 166 mg of the powdered material was added to the vial and a timer was started. The crosslink time was measured as the time when the stir bar stopped due to the formation of the crosslinked hydrogel. The crosslink time was measured in both saline and recalcified citrated whole sheep's blood (631 microliters of blood and 33 microliters of 0.2 M CaCl₂). The measured crosslink time of the material was faster in blood than in saline at 37 °C, as shown in Table 5.

**Table 5: Measured crosslink time of a PEG(SS)2 dry powder material in blood and saline.**

| | **Crosslink time in blood (sec)** | **Crosslink time in saline (sec)** |
|---|---|---|
| Average (n=5) | 44.4 | 76.6 |
| Standard deviation (n=5) | 6.9 | 15.8 |

### EXAMPLE 8

The following example describes the effect of the amount of base on the measured crosslink time of a PEG(SS)2 dry powder composition. The dry powder material was prepared by mixing PEG(SS)2 (119 mg), bovine serum albumin (BSA) (228 mg) and various amounts of sodium bicarbonate and/or calcium chloride (CaCl₂). The calcium chloride was used in order to keep the composition ratios fixed, including the salt concentration. The measured crosslink time was measured as described in Example 7, and the resulting pH of the hydrogel was measured with a surface electrode. The results in Table 6 show the importance of sodium bicarbonate content on obtaining a basic pH and fast measured crosslink time.

**Table 6: Effect of the amount of base on the crosslink time of a dry powder material.**

| **Weight % Sodium bicarbonate** | **Weight % Calcium chloride** | **Crosslink time in blood (mean ± std. dev.) (sec)** | **pH in blood (mean ± std. dev.)** | **Crosslink time in saline (mean ± std. dev.) (sec)** | **pH in saline (mean ± std. dev.)** |
|---|---|---|---|---|---|
| 30 | 0 | 56.6 ± 12.5 | 9.2 ± 0.2 | 83.6 ± 17.7 | 8.7 ± 0.1 |
| 22.5 | 7.5 | 112.7 ± 41.2 | 8.6 ± 0.3 | 244.5 ± 27.4 | 7.7 ± 0.6 |
| 15 | 15 | 136.6 ± 45.6 | 8.1 ± 0.4 | 798.4 ± 79.9 | 7.0 ± 0.3 |
| 7.5 | 22.5 | 141.4 ± 44.5 | 7.4 ± 0.3 | 1058.8 ±179.3 | 6.8 ± 0.1 |
| 0 | 30 | 133.8 ± 10.0 | 6.2 ± 0.4 | 8253.3 ± 665.8 | 4.9 ± 0.1 |

### EXAMPLE 9

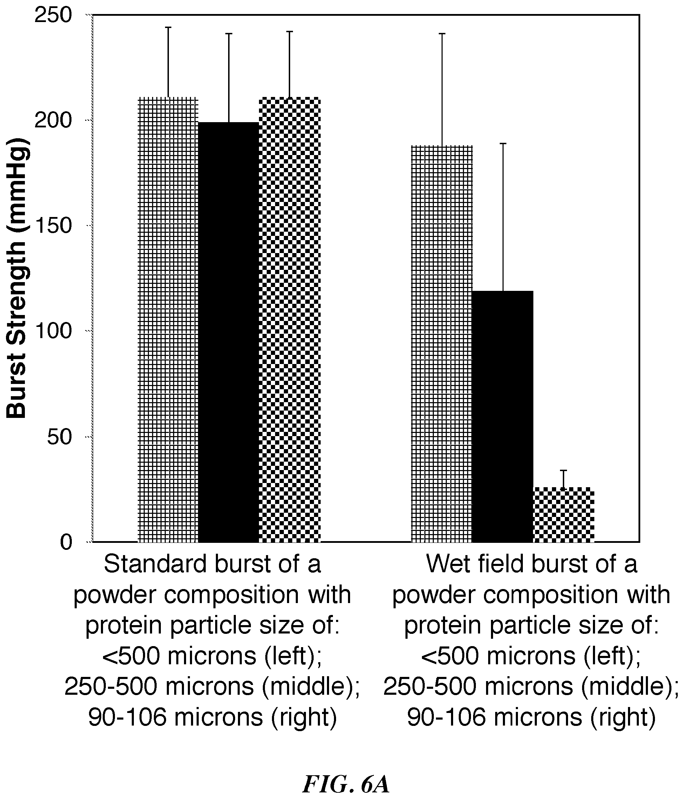

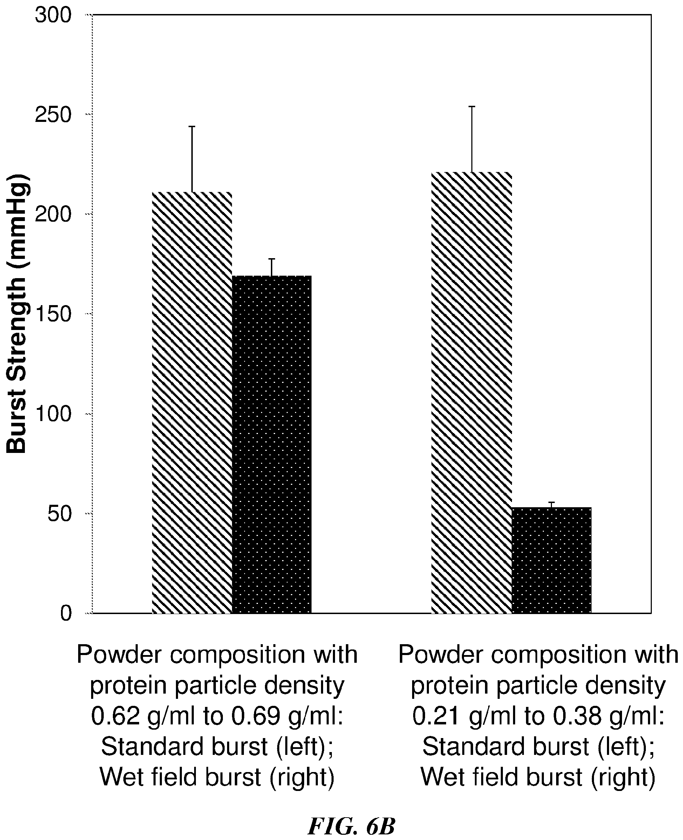

The following example describes the effect of protein particle size and protein particle density on the measured crosslink time of a dry powder composition. The material was prepared as in Example 1, but with BSA of different particle sizes and particle densities. The BSA with different particle sizes were obtained by sieving the starting BSA powder. The particle density of the BSA powder was measured by the tapped density method described previously and ranged from 0.62 g/ml to 0.69 g/ml. Lower density protein particles were made by dissolving the starting BSA powder at concentrations of 30% w/v and 7.5% w/v in deionized water, followed by lyophilizing the solutions. The resulting tapped densities of the lyophilized BSA powders were 0.38 g/ml and 0.21 g/ml for the 30% w/v and 7.5% w/v solutions, respectively. The reactive powders were tested in the standard burst strength model and the modified wet field burst strength model as described in Example 4 and Example 6, respectively. Measured crosslink time in blood and saline was also determined as described in Example 8. The results indicated that smaller particle sizes and lower particle densities had reduced performance in the wet field burst strength model that was not evident in the standard model, as shown in FIG. 6A and FIG. 6B. In addition, the mid-range particle sizes displayed the fastest measured crosslink time, as shown in Table 7.

**Table 7: Effect of the particle size on the crosslink time of a dry powder material.**

| **Albumin particle size (microns)** | **Average (n=3) crosslink time in blood (sec)** | **Standard deviation (n=3) crosslink time in blood (sec)** | **Average (n=3) crosslink time in saline (sec)** | **Standard deviation (n=3) crosslink time in saline (sec)** |
|---|---|---|---|---|
| 250-500 | 56.88 | 3.12 | 94.10 | 7.00 |
| 106-250 | 36.94 | 4.37 | 55.82 | 1.73 |
| <106 | 63.15 | 1.42 | 202.68 | 70.33 |
| 1-500 | 44.2 | 6.87 | 76.58 | 15.79 |

### EXAMPLE 10

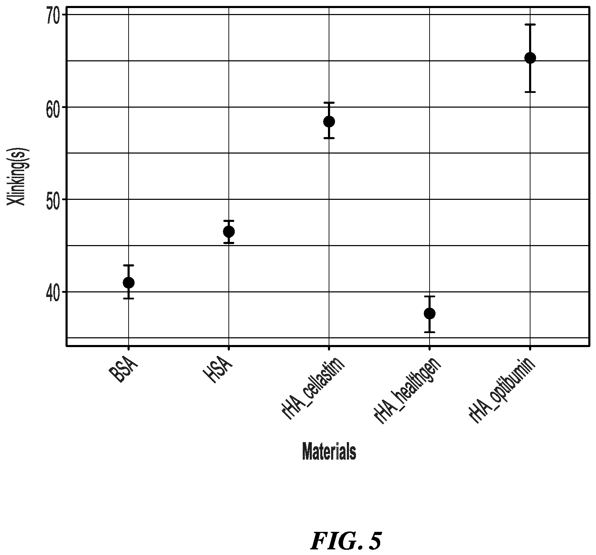

The following example describes the effect of using to various types of albumin on the measured crosslink time (determined as in Example 8) of a dry powder material. The material was prepared as in Example 1, but with albumin from different sources, including bovine serum albumin, human serum albumin, and various sources of recombinant human albumin. All of the varieties of albumin used in the reactive powder material crosslinked into tissue adherent hydrogels. The measured crosslink time in blood of the mixtures varied, as shown in FIG. 5, which may be due to the various particle sizes and/or densities of the albumin.

### EXAMPLE 11

The following example describes a comparison of various hydrogel properties resulting from altering the ratio of components of the dry powder mixture. Various dry powder mixtures (i.e., Samples) were formulated as shown in Table 8. The bovine serum albumin (BSA) and PEG(SS)2 particles were sieved to remove particles with a particle size greater than 500 micrometers.

**Table 8: Amounts of components of a dry, powdered mixture.**

| **Sample** | **Amount of BSA (g)** | **Amount of PEG(SS)2 (g)** | **Amount of NaHCO₃ (g)** | **BSA/PEG(SS)2 Mass Ratio** |
|---|---|---|---|---|
| 1 | 0.5696 | 0.270 | 0.1604 | 2.110 |
| 2 | 0.6098 | 0.305 | 0.0852 | 1.999 |
| 3 | 0.6148 | 0.300 | 0.0852 | 2.049 |
| 4 (30 wt.% NaHCO₃) | 0.4560 | 0.238 | 0.3060 | 1.916 |
| 5 | 0.6600 | 0.170 | 0.1700 | 3.882 |
| 6 | 0.4618 | 0.305 | 0.2332 | 1.514 |
| 7 (1 wt.% NaHCO₃) | 0.6500 | 0.340 | 0.0100 | 1.912 |
| 8 | 0.3540 | 0.340 | 0.3060 | 1.041 |
| 9 | 0.5468 | 0.220 | 0.2332 | 2.485 |
| 10 (3 wt.% NaHCO₃) | 0.6370 | 0.333 | 0.0300 | 1.913 |
| 11 | 0.6148 | 0.220 | 0.1652 | 2.795 |

The experimental values reported for each parameter discussed below and shown in Tables 9 and 10 are an average of multiple trials (n), as indicated in each table.

The measured crosslink time of each sample was evaluated as described herein. The measured crosslink time of samples 1-6, 8, 9, and 11 were each less than 120 seconds. Samples 7 and 10, with low sodium bicarbonate levels, had crosslink times that were greater than the other samples (i.e., ~300 seconds and ~120 seconds, respectively).

The standard burst strength of each sample was evaluated. Sample 7 had adhesive failures and a significantly lower burst strength (i.e., ~50 mm Hg) as compared to the other samples, each of which had mean burst strengths between greater than 100 mm Hg and less than 200 mm Hg.

An ElastoSens^{™} Bio² instrument was used to evaluate the viscoelastic properties of the samples. The maximum shear elastic modulus (G' max) was evaluated for each sample. Sample 7 exhibited a significantly higher G' max (i.e., ~20000 Pa) as compared to the other samples, each of which were between greater than 7500 Pa and less than 15000 Pa. The results generally correlated with the amount of sodium bicarbonate in the sample, such that samples with less sodium bicarbonate (and more BSA and PEG(SS)2) exhibited a higher G' max.

The maximum gelation rate (max gelation rate) was also evaluated using the ElastoSens^{™} Bio² instrument. Samples 7 and 8 had significantly lower max gelation rates (i.e., less than 100 Pa/sec) as compared to the other samples, each of which has a max gelation rate between greater than 125 Pa/sec and less than 325 Pa/sec. Samples 9 and 11, both with a high BSA/PEG(SS)2 ratio, had max gelation rates that were significantly higher than the other groups (i.e., ~270 Pa/sec and ~325 Pa/sec, respectively).

The time of maximum gelation rate (time of max gelation rate) was also evaluated using the ElastoSens^{™} Bio² instrument. This parameter is the time at which the maximum rate of hydrogel formation occurred. Samples 7 and 10, with low sodium bicarbonate levels, had times of max gelation rates that were significantly slower (i.e., ~62 seconds and ~50 seconds, respectively), than the other samples (i.e., less than 42 seconds). The results generally correlated with the amount of sodium bicarbonate in the sample, such that samples with less sodium bicarbonate took longer to reach the max gelation rate.

The time to reach a G' of 5000 Pa was also evaluated using the ElastoSens^{™} Bio² instrument. This parameter is the time at which the hemostatic hydrogel has a shear elastic modulus 4-5 times higher than a blood clot. Samples 7 and 8 took longer (e.g., greater than 75 seconds) to reach a G' of 5000 Pa as compared to the other samples, each of which took less than 55 seconds to reach a G' of 5000 Pa. Sample 11 had the shortest time to reach a G' of 5000 Pa (~23 seconds).

The shear elastic modulus (G') at 150 seconds was also evaluated using the ElastoSens^{™} Bio² instrument. The 150 second time mark was chosen because it is the time allowed for the hydrogel to cure in the west burst test method. Sample 8 had a low G' at 150 seconds (i.e., ~3500 Pa), while sample 11 had a higher G' at 150 seconds (i.e. ~11500 Pa).

The surface pH of the samples was also measured. Samples 7 and 10, with low sodium bicarbonate levels, had surface pH values (i.e., ~6.5 and ~7, respectively) that were lower than the other groups, each of which had a surface pH of greater than ~8.

**Table 9: Experimental values obtained using the formulations in Table 8.**

| **Sample** | **Measured crosslink time (mean ± std. dev.) (sec)** | **Standard burst strength (mean ± std. dev.) (mm Hg)** | **Surface pH** |
|---|---|---|---|
| 1 | 76 ± 10 (n=10) | 153 ± 50 (n=10) | 8.2 ± 0.4 (n=5) |
| 2 | 90 ± 6 (n=10) | 150 ± 46 (n=10) | 8.3 ± 0.4 (n=5) |
| 3 | 93 ± 14 (n=10) | 185 ± 42 (n=10) | 8.1 ± 0.6 (n=5) |
| 4 (30 wt.% NaHCO₃) | 79 ± 13 (n=10) | 191 ± 35 (n=10) | 8.5 ± 0.2 (n=10) |
| 5 | 75 ± 9 (n=10) | 186 ± 46 (n=10) | 8.4 ± 0.2 (n=10) |
| 6 | 76 ± 14 (n=10) | 147 ± 54 (n=10) | 8.4 ± 0.2 (n=10) |
| 7 (1 wt.% NaHCO₃) | 317 ± 81 (n=10) | 44 ± 23 (n=10) | 6.4 ± 0.3 (n=10) |
| 8 | 107 ± 42 (n=10) | 112 ± 53 (n=10) | 8.8 ± 0.2 (n=10) |
| 9 | 82 ± 16 (n=10) | 169 ± 30 (n=10) | 8.6 ± 0.2 (n=10) |
| 10 (3 wt.% NaHCO₃) | 135 ± 32 (n=10) | 149 ± 47 (n=10) | 7.0 ± 0.4 (n=10) |
| 11 | 74 ± 9 (n=10) | 194 ± 30 (n=10) | 8.3 ± 0.2 (n=10) |

**Table 10: ElastoSens^{™} Bio² experimental values determined using the formulations in Table 8.**

| **Sample** | **G' Max (mean ± std. dev.) (Pa)** | **Max gelation rate (mean ± std. dev.) (Pa/sec)** | **Time of max gelation rate (mean ± std. dev.) (sec)** | **Time to reach G' of 5000 Pa (mean ± std. dev.) (sec)** | **G' at 150 seconds (mean ± std. dev.) (Pa)** |
|---|---|---|---|---|---|
| 1 | 11455 ± 2565 (n=10) | 168 ± 17 (n=10) | 34 ± 5 (n=10) | 50 ± 10 (n=10) | 7477 ± 1631 (n=10) |
| 2 | 12997 ± 1668 (n=5) | 186 ± 24 (n=6) | 33 ± 6 (n=5) | 40 ± 7 (n=4) | 8414 ± 1559 (n=5) |
| 3 | 14998 ± 1252 (n=9) | 190 ± 18 (n=9) | 41 ± 7 (n=9) | 39 ± 3 (n=8) | 9037 ± 1385 (n=10) |
| 4 (30 wt.% NaHCO₃) | 8517 ± 2132 (n=10) | 157 ± 42 (n=10) | 33 ± 8 (n=10) | 40 ± 6 (n=9) | 6316 ± 1624 (n=10) |
| 5 | 8099 ± 1104 (n=9) | 212 ± 53 (n=10) | 30 ± 6 (n=10) | 33 ± 13 (n=10) | 7184 ± 1296 (n=9) |
| 6 | 11527 ± 1635 (n=10) | 169 ± 42 (n=10) | 35 ± 8 (n=10) | 46 ± 10 (n=8) | 7014 ± 1612 (n=10) |
| 7 (1 wt.% NaHCO₃) | 20419 ± 2987 (n=10) | 88 ± 15 (n=10) | 63 ± 10 (n=10) | 74 ± 9 (n=10) | 9857 ± 534 (n=9) |
| 8 | 9180 ± 2309 (n=10) | 95 ± 10 (n=10) | 37 ± 5 (n=10) | 257 ± 75 (n=10) | 3833 ± 760 (n=10) |
| 9 | 10779 ± 1993 (n=10) | 263 ± 31 (n=10) | 31 ± 3 (n=10) | 30 ± 5 (n=10) | 8909 ± 1749 (n=10) |
| 10 (3 wt.% NaHCO₃) | 15357 ± 1580 (n=10) | 145 ± 24 (n=9) | 51 ± 12 (n=9) | 53 ± 7 (n=9) | 7895 ± 487 (n=9) |
| 11 | 13591 ± 1689 (n=10) | 327 ± 34 (n=10) | 27 ± 3 (n=10) | 24 ± 3 (n=10) | 11549 ± 1192 (n=10) |

### EXAMPLE 12

The following example describes a comparison of various hydrogel properties resulting from altering the ratio of components of the dry powder mixture. Various dry powder mixtures (i.e., Samples) were formulated based on the amounts shown in Table 11. The bovine serum albumin (BSA) particles were sieved to remove particles with a particle size greater than 500 micrometers.

**Table 11: Relative amounts of components of a dry, powdered mixture.**

| **Sample** | **Amount of BSA (g)** | **Amount of PEG(SS)2 (g)** | **Amount of NaHCO₃ (g)** | **BSA/PEG(SS)2 mass ratio** |
|---|---|---|---|---|
| 1 | 0.6600 | 0.3100 | 0.030 | 2.13 |
| 2 | 0.6375 | 0.2875 | 0.075 | 2.22 |
| 3 | 0.6500 | 0.2600 | 0.090 | 2.50 |
| 4 | 0.6100 | 0.2400 | 0.150 | 2.54 |
| 5 | 0.6150 | 0.2650 | 0.120 | 2.32 |
| 6 | 0.6150 | 0.2200 | 0.165 | 2.80 |
| 7 | 0.6700 | 0.2700 | 0.060 | 2.48 |

The experimental values reported for each parameter discussed below and shown in Tables 12 and 13 are an average of multiple trials (n), as indicated in each table.

The measured crosslink time of each sample was evaluated as described herein. The measured crosslink time of samples 1 and 7, which had the lowest amounts of sodium bicarbonate, were ~168 seconds and 99 seconds, respectively. The additional samples had a measured crosslink time between less than 90 seconds and greater than 60 seconds.

The wet field burst strength was evaluated. All samples exhibited a mean wet field burst strength between greater than 130 mm Hg and less than 210 mm Hg.

An ElastoSens^{™} Bio² instrument was used to evaluate the viscoelastic properties of the samples. The maximum shear elastic modulus (G' max) was evaluated for each sample. Sample 6 had the largest amount of sodium bicarbonate (16.5 wt.%) and the lowest G' max value (~14000 Pa). The G' max of the other samples ranged from less than 18500 Pa to greater than 14900 Pa.

The maximum gelation rate (max gelation rate) was also evaluated using the ElastoSens^{™} Bio² instrument. Samples 1 and 7, which had the lowest amounts of sodium bicarbonate, had lower max gelation rates (i.e., ~125 Pa/sec and ~145 Pa/sec, respectively). The max gelation rates of the other samples were between less than 235 Pa/sec and greater than 195 Pa/sec.

The time of maximum gelation rate (time of max gelation rate) was also evaluated using the ElastoSens^{™} Bio² instrument. Samples 1 and 7 had greater times of max gelation rate (each ~52 seconds) as compared to the other samples, each of which had a time of max gelation rate between greater than 32 second and less than 41 seconds.

The time to reach a G' of 5000 Pa was also evaluated using the ElastoSens^{™} Bio² instrument. Samples 1 and 7 had the longest times to reach a G' of 5000 Pa at ~56 seconds and ~54 seconds, respectively. The other samples took between 32 seconds and 39 seconds to reach a G' of 5000 Pa.

The shear elastic modulus (G') at 150 seconds was also evaluated using the ElastoSens^{™} Bio² instrument. Samples 1 and 7 had the lowest G' at 150 seconds (i.e., ~11450 Pa and ~10350 Pa, respectively). The G' at 150 seconds for samples 2-6 was between less than 13800 Pa and greater than 11700 Pa.

**Table 12: Experimental values obtained using the formulations in Table 11.**

| **Sample** | **Measured crosslink time (mean ± std. dev.) (sec)** | **Wet field burst strength (mean ± std. dev.) (mm Hg)** |
|---|---|---|
| 1 | 168 ± 41 (n=5) | 149 ± 77 (n=10) |
| 2 | 89 ± 11 (n=5) | 201 ± 55 (n=10) |
| 3 | 85 ± 17 (n=5) | 155 ± 66 (n=10) |
| 4 | 79 ± 10 (n=5) | 142 ± 71 (n=10) |
| 5 | 64 ± 4 (n=5) | 189 ± 62 (n=10) |
| 6 | 63 ± 4 (n=5) | 184 ± 73 (n=10) |
| 7 | 99 ± 10 (n=5) | 154 ± 43 (n=10) |

**Table 13: ElastoSens^{™} Bio² experimental values determined using the formulations in Table 11.**

| **Sample** | **G' Max (mean ± std. dev.) (Pa)** | **Max gelation rate (mean ± std. dev.) (Pa/sec)** | **Time of max gelation rate (mean ± std. dev.) (sec)** | **Time to reach G' of 5000 Pa (mean ± std. dev.) (sec)** | **G' at 150 seconds (mean ± std. dev.) (Pa)** |
|---|---|---|---|---|---|
| 1 | 17708 ± 2026 (n=12) | 126 ± 15 (n=12) | 52 ± 12 (n=12) | 56 ± 9 (n=12) | 11449 ± 1801 (n=12) |
| 2 | 18476 ± 1821 (n=12) | 211 ± 26 (n=12) | 40 ± 5 (n=12) | 38 ± 5 (n=12) | 13743 ± 1374 (n=12) |
| 3 | 17128 ± 1313 (n=12) | 197 ± 34 (n=12) | 39 ± 6 (n=12) | 39 ± 7 (n=12) | 13156 ± 1360 (n=12) |
| 4 | 15066 ± 1089 (n=12) | 232 ± 25 (n=12) | 33 ± 3 (n=12) | 32 ± 3 (n=12) | 12867 ± 797 (n=12) |
| 5 | 17546 ± 1271 (n=12) | 217 ± 27 (n=12) | 40 ± 5 (n=12) | 39 ± 5 (n=12) | 13619 ± 1141 (n=11) |
| 6 | 14116 ± 893 (n=12) | 218 ± 28 (n=12) | 38 ± 5 (n=12) | 36 ± 4 (n=12) | 11760 ± 1071 (n=12) |
| 7 | 14972 ± 1374 (n=12) | 145 ± 22 (n=12) | 52 ± 13 (n=12) | 54 ± 11 (n=12) | 10360 ± 1286 (n=12) |

### EXAMPLE 13

The following example describes a comparison of various hydrogel properties resulting from altering the density and particle size of human serum albumin (HSA) in the dry powdered mixture. Dry powder mixtures (i.e., Samples) were prepared using HSA with various combinations of density and particle size as shown in Table 14. The "low" density groups used the starting HSA powder (e.g., less than 0.4 g/ml) and the "high" density group used the rolled compacted HSA powder (greater than 0.5 g/ml). The "low", "medium", and "high" particle size groups correspond to HSA powder sieved to particle size targets of less than 106 micrometers, between 106 and 250 micrometers, and between 250 and 500 micrometers, respectively.

**Table 14: Relative amounts of components of a dry, powdered mixture.**

| **Sample** | **Density** | **Particle Size** | **HSA Particle Size Targets (micrometers)** |
|---|---|---|---|
| 1 | Low | Low | <106 |
| 2 | Low | Medium | 106-250 |
| 3 | Low | High | 250-500 |
| 4 | High | Low | <106 |
| 5 | High | Medium | 106-250 |
| 6 | High | High | 250-500 |
| 7 | Low | Full Range | 1:1:1 blend of the 3 sizes |
| 8 | High | Full Range | 1:1:1 blend of the 3 sizes |

The experimental values reported for each parameter listed below and shown in Tables 15 and 16 are an average of multiple trials (n), as indicated in each table.

The measured crosslink time of each sample was evaluated as described herein. Sample 1, with low density and smaller sized HSA particles, sat on the surface of the liquid and no crosslinking was observed within 180 seconds. Samples 2 and 3, with low density particles that were larger than the particles in Sample 1, crosslinked in less than 60 seconds. The particle sizes in Samples 2 and 3 were observed to better penetrate the liquid surface, as compared to Sample 1, and because the particles were low density they dissolved quickly to participate in the crosslinking reaction. Sample 4 also crosslinked in less than 60 seconds. Samples 6, 7, and 8 each had crosslink times greater than 100 seconds, presumably due to the higher density of the particles, which solubilized slower than the low density particles.

The wet field burst strength of each sample was evaluated. All samples exhibited a mean wet field burst strength between 90 and 200 mm Hg.

An ElastoSens^{™} Bio² instrument was used to evaluate the viscoelastic properties of the samples. The maximum shear elastic modulus (G' max) was evaluated for each sample. The G' max of Sample 6 (e.g., less than 10000 Pa) was lower than the other groups, each of which had a G' max between greater than 12500 Pa and less than 21000 Pa.

The maximum gelation rate (max gelation rate) was also evaluated using the ElastoSens^{™} Bio² instrument. Sample 6 had the lowest max gelation rate (less than 100 Pa/sec). The other samples had max gelation rates between greater than 200 Pa/sec and less than 700 Pa/sec.

The time of maximum gelation rate (time of max gelation rate) was also evaluated using the ElastoSens^{™} Bio² instrument. Samples 1 and 4, with HSA particles less than 106 micrometers, had longer times of max gelation rates (~53 seconds and ~63 seconds, respectively), as compared to the other samples, each of which has a time of max gelation rate between less than 30 seconds and greater than 15 seconds.

The time to reach a G' of 5000 Pa was also evaluated using the ElastoSens^{™} Bio² instrument. Sample 6 had the longest time to reach a G' of 5000 Pa (~90 seconds), presumably due to the slower solubility of the larger, density HSA particles. Samples 1 and 4 both took greater than 40 seconds to reach a G' of 5000 Pa, presumably due to the small particle size. Samples 2, 3, 5, 7, and 8 reached a G' of 5000 Pa in less than 20 seconds.

The shear elastic modulus (G') at 150 seconds was also evaluated using the ElastoSens^{™} Bio² instrument. Samples 4 and 6 had a shear elastic modulus less than 10000 Pa at 150 seconds, while the other samples had a shear elastic modulus between greater than 12500 Pa and less than 17500 Pa at 150 seconds.

**Table 15: Experimental values obtained using the formulations in Table 14.**

| **Sample** | **Measured crosslink time (mean ± std. dev.) (sec)** | **Wet field burst strength (mean ± std. dev.) (mm Hg)** |
|---|---|---|
| 1 | ≥180 (n=10) | 117 ± 78 (n=10) |
| 2 | 48 ± 3 (n=10) | 163 ± 41 (n=10) |
| 3 | 56 ± 4 (n=10) | 190 ± 53 (n=10) |
| 4 | 55 ± 3 (n=10) | 90 ± 58 (n=10) |
| 5 | 67 ± 6 (n=10) | 140 ± 69 (n=10) |
| 6 | 120 ± 28 (n=10) | 160 ± 54 (n=10) |
| 7 | 95 ± 25 (n=10) | 165 ± 61 (n=10) |
| 8 | 158 ± 36 (n=10) | 173 ± 59 (n=10) |

**Table 16: ElastoSens^{™} Bio² experimental values determined using the formulations in Table 14.**

| **Sample** | **G' Max (mean ± std. dev.) (Pa)** | **Max gelation rate (mean ± std. dev.) (Pa/sec)** | **Time of max gelation rate (mean ± std. dev.) (sec)** | **Time to reach G' of 5000 Pa (mean ± std. dev.) (sec)** | **G' at 150 seconds (mean ± std. dev.) (Pa)** |
|---|---|---|---|---|---|
| 1 | 15729 ± 1878 (n=8) | 326 ± 97 (n=7) | 54 ± 9 (n=7) | 47 ± 6 (n=8) | 14655 ± 1501 (n=8) |
| 2 | 16075 ± 1760 (n=9) | 482 ± 90 (n=10) | 17 ± 3 (n=10) | 15 ± 3 (n=10) | 14862 ± 1380 (n=10) |
| 3 | 14901 ± 1359 (n=10) | 206 ± 22 (n=10) | 30 ± 7 (n=10) | 34 ± 4 (n=10) | 12782 ± 973 (n=10) |
| 4 | 13057 + 2304 (n=9) | 277 ± 109 (n=9) | 63 ± 14 (n=10) | 52 ± 17 (n=9) | 9660 ± 1839 (n=9) |
| 5 | 14837 ± 1770 (n=10) | 344 ± 45 (n=10) | 21 ± 4 (n=10) | 19 ± 4 (n=10) | 13577 ± 1292 (n=10) |
| 6 | 9079 ± 1008 (n=10) | 73 ± 12 (n=10) | 18 ± 7 (n=10) | 94 ± 10 (n=10) | 6963 ± 821 (n=10) |
| 7 | 16748 ± 2004 (n=10) | 407 ± 94 (n=10) | 23 ± 4 (n=10) | 21 ± 4 (n=10) | 14907 ± 1337 (n=10) |
| 8 | 20949 ± 3906 (n=9) | 661 ± 100 (n=10) | 24 ± 6 (n=10) | 21 ± 5 (n=10) | 17321 ± 2656 (n=9) |

### EXAMPLE 14

The following example describes the standard burst strength of a crosslinked hydrogel formed from a dry powdered composition at 0 hours and 24 hours. The dry powdered composition contained 24 wt.% PEG(SS)2, 61 wt.% bovine serum albumin (BSA), and 15 wt.% sodium bicarbonate. Samples were prepared for burst testing as described previously. Half of the samples were tested at t=0 (e.g., the time of hydration) and half were removed from the burst fixture and placed in phosphate buffered saline (PBS) at 37°C. The PBS was replaced after 1 hour to maintain neutral pH. After 24 hours, the samples were removed from the PBS still attached to the collagen substrate, returned to the fixture and burst tested. The data shown below in Table 17 indicated that the hemostatic hydrogel maintained sufficient burst strength in vitro for 24 hours.

**Table 17: Burst strength of a dry powdered hemostatic composition at 0 and 24 hours.**

| **Time (hours)** | **Sample size** | **Max Burst Strength (mean ± std. dev.) (mm Hg)** | **Burst Strength Range (mm Hg)** |
|---|---|---|---|
| 0 | 10 | 201 ± 50 | 92-255 |
| 24 | 9 | 152 ± 18 | 118 - 183 |

### EXAMPLE 15

The following example describes the hemostatic efficacy of various dry powder mixtures in a porcine spleen biopsy defect model. Various dry powder mixtures (i.e., Samples) were formulated as shown in Table 18.

**Table 18: Various formulations of dry powder mixtures used to evaluate the hemostatic efficiency in a porcine spleen biopsy defect model.**

| **Sample** | **Formulation** | **Wt. % Albumin** | **Wt. % PEG(SS)2** | **Wt. % Unmodified PEG** | **Wt. % NaHCO₃** |
|---|---|---|---|---|---|
| 1 | PEG(SS)2 w/ BSA | 61 | 24 | 0 | 15 |
| 2 | PEG(SS)2 w/ HSA | 61 | 24 | 0 | 15 |
| 3 | 70% functional PEG(SS)2 | 61 | 16.8 | 7.2 | 15 |
| 4 | 50 wt.% functional PEG(SS)2 | 61 | 12 | 12 | 15 |
| 5 | PEG(SS)2 w/ spray coated HSA/SB | 61 | 24 | 0 | 15 |
| 6 | 85 wt.% functional PEG(SS)2 | 61 | 20.4 | 3.6 | 15 |
| 7 | 120 wt.% functional PEG(SS)2 | 56 | 29 | 0 | 15 |

Heparin was administered to each animal to mimic clinically relevant coagulopathies and heparinization during cardiovascular surgery. An IV bolus of heparin was given initially to increase the activated clotting time (ACT) to ≥ 2-3× baseline and periodically thereafter for maintenance. In order to increase the hemostatic challenge in a realistic surgical scenario, the dry powdered material was applied to an actively bleeding site, instead of blotting the defect dry prior to placement. A 10 mm diameter biopsy defect to a depth of ~5 mm was made in the spleen. The defect was allowed to fill with blood and then the sample powder mixture (0.5 g) was applied to the defect and a 2.5-3 cm × 2.5-3 cm starch foam or carboxymethylcellulose (CMC) tamponade was placed on top of the powder to prevent it from adhering to the gauze used for application of the tamponade. Initial hemostasis was assessed after each 10 second tamponade pressure application cycle for a duration of 30 seconds. If the defect remained hemostatic for the duration of the 30 second observation, it was declared initially hemostatic. If the defect was still bleeding, additional tamponade cycles were applied. If hemostasis was achieved for 30 seconds after any of the tamponade cycles, the defect was observed for an additional 5 minutes to check for rebleeding. Results are shown in Table 19.

**Table 19. Hemostatic efficacy of various dry powder mixtures in a heparinized porcine spleen abrasion model.**

| **Sample** | **Formulation** | **# of Defects Treated** | **# of Pressure Cycles (mean ± std. dev.)** | **% of Defects Achieving and Maintaining Hemostasis (5 min Observation)** |
|---|---|---|---|---|
| 1 | PEG(SS)2 w/ BSA | 12 | 1.4 ± 1.2 | 92 |
| 2 | PEG(SS)3 w/ HSA | 12 | 1.3 ± 0.7 | 92 |
| 3 | 70% functional PEG(SS)2 | 12 | 2.2 ± 2.2 | 83 |
| 4 | 50 wt.% functional PEG(SS)2 | 12 | 5.1 ± 4.4 | 50^ |
| 5 | PEG(SS)2 w/ spray coated HSA/SB | 12 | 1.9 ± 1.1 | 92 |
| 6 | 85 wt.% functional PEG(SS)2 | 12 | 2.6 ± 3.3 | 83 |
| 7 | 120 wt.% functional PEG(SS)2 | 12 | 2.1 ± 2.5 | 92 |
| Control | Floseal | 8 | 8.9 ± 2.2 | 0 |

### EXAMPLE 16

The following example describes the hemostatic efficacy of dry powder mixtures comprising albumin of different particle sizes in a porcine spleen biopsy defect model. Samples were prepared and evaluated as explained in Example 15. Smaller album particles ranged from 54 to 299 micrometers, while larger albumin particles ranges from 101 to 547 micrometers. The data tabulated in Table 20 shows that smaller particles generally have a negative effect on hemostatic efficiency.

**Table 20: Hemostatic efficacy of various dry powder mixtures in a heparinized porcine spleen abrasion model.**

| **Formulation** | **# of Defects Treated** | **# of Pressure Cycles (mean ± std. dev.)** | **% of Defects Achieving Hemostasis in One Pressure Cycle** |
|---|---|---|---|
| Dry powder mixture w/ smaller albumin particles | 5 | 2.0 ± 1.7 | 60 |
| Dry powder mixture w/ larger albumin particles | 5 | 1.0 ± 0.0 | 100 |

### EXAMPLE 17

The following example describes the hemostatic efficacy of dry powder mixtures comprising varying amounts of sodium bicarbonate in a porcine spleen biopsy defect model. Samples were prepared and evaluated as explained in Example 15. The data tabulated in Table 21 show that lower sodium bicarbonate levels generally have a negative effect on hemostatic efficacy.

**Table 21. Hemostatic efficacy of various dry powder mixtures comprising different amounts of sodium bicarbonate in a heparinized porcine spleen abrasion model.**

| **Sample** | **# of Defects Treated** | **# of Pressure Cycles (mean ± std. dev.)** | **% of Defects Achieving Hemostasis in One Pressure Cycle** |
|---|---|---|---|
| Dry powder mixture with 1 wt.% NaHCO₃ (30 second pressure cycles) | 6 | 2.3 ± 1.4 | 33 |
| Dry powder mixture with 3 wt.% NaHCO₃ (30 second pressure cycles) | 6 | 1.3 ± 0.5 | 67 |
| Dry powder mixture with 16.5 wt.% NaHCO₃ (30 second pressure cycles) | 5 | 1.8 ± 1.8 | 80 |
| Dry powder mixture with 30 wt.% NaHCO₃ (30 second pressure cycles) | 3 | 1.0 ± 0.0 | 100 |
| Dry powder mixture with 16.5 wt.% NaHCO₃ (30 second pressure cycles) | 4 | 2.3 ± 2.5 | 75 |

### EXAMPLE 18

The following example describes the effect of various fluids used to hydrate the dry powdered mixture on hydrogel formation. A dry powdered mixture was prepared with 24 wt.% PEG(SS)2, 61 wt.% albumin, and 15 wt.% sodium bicarbonate. The dry powder mixture was hydrated with the fluids shown in Table 22. The pH and viscoelastic properties were then evaluated (where applicable).

**Table 22. Various fluids used to hydrate a dry powdered mixture and the resulting experimental parameters.**

| **Sample** | **Description** | **pH** | **G' Max (Pa)** | **G' at 150 seconds (Pa)** | **Time to reach G' of 5000 Pa (sec)** |
|---|---|---|---|---|---|
| Reference | Citrated blood | Not | 1276 | 0 | N/A |
| | with CaCl₂ without added dry powdered mixture | evaluated | | | |
| 1 | Dry powdered mixture + PB S | 7.2-7.4 | 12986 | 10267 | 31 |
| 2 | Dry powdered mixture + 0.03 M HCl | 1.5 | 13467 | 7041 | 67 |
| 3 | Dry powdered mixture + Citrated blood | 7.35-7.45 | 42990 | 40057 | 9 |
| 4 | Dry powdered mixture + Citrated blood with CaCl₂ | Not evaluated | 51450 | 46046 | immediate |

The dry powder mixture was able to hydrate and quickly crosslink in all four fluids to form a simulated hydrogel clot with a higher modulus than a reference blood clot. The lower strength clots were formed from the dry powder mixture hydrated with PBS or a very acidic simulated gastric fluid (0.03 M hydrochloric acid) and the clot strength in both fluids were still at least 9-10 times higher than a reference blood clot. The clots formed by polymerizing the dry powder mixture with citrated blood or citrated blood with calcium chloride were much higher in strength with a modulus of 30-40 times that of a blood clot, supporting that the dry powder mixture can function in a variety of coagulopathic situations.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified unless clearly indicated to the contrary. Thus, as a non-limiting example, a reference to "A and/or B," when used in conjunction with open-ended language such as "comprising" can refer, in one embodiment, to A without B (optionally including elements other than B); in another embodiment, to B without A (optionally including elements other than A); in yet another embodiment, to both A and B (optionally including other elements); etc. As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

In the claims, as well as in the specification above, all transitional phrases such as "comprising," "including," "carrying," "having," "containing," "involving," "holding," and the like are to be understood to be open-ended, i.e., to mean including but not limited to. Only the transitional phrases "consisting of" and "consisting essentially of" shall be closed or semi-closed transitional phrases, respectively.

## Claims

1. A dry, powdered, crosslinking hemostatic composition, comprising:
a first component comprising a difunctionalized polyalkylene oxide-based component of any of the forms: 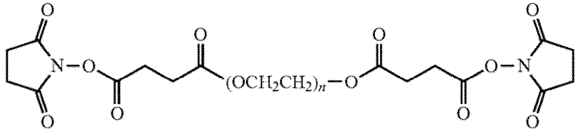 Poly(ethylene glycol) disuccinimidoyl succinate 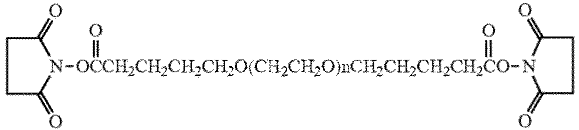 PEG disuccinimidyl valerate 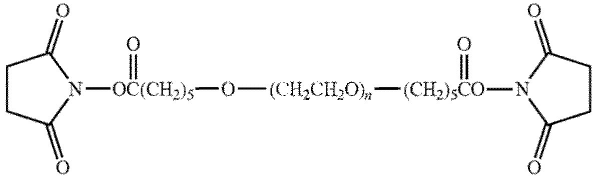 PEG disuccinimidyl hexanoate; and
a second component comprising:
a protein that is capable of crosslinking with the first component; and
a crosslinking initiator that initiates crosslinking of the first component with the protein, wherein the crosslinking initiator comprises a base and/or a basic buffer, wherein the base and/or basic buffer comprises sodium bicarbonate and/or a base that does not include amine functionality;
wherein upon exposure to an aqueous liquid, crosslinking is initiated to form a hemostatic hydrogel.

2. The composition of claim 1, wherein the protein is selected from the group consisting of: human serum albumin, recombinant human albumin, and animal sourced albumin.

3. The composition of any one of claims 1 or 2, wherein the composition comprises at least a first dry powder and a second dry powder.

4. The composition of claim 3, wherein the first dry powder comprises the first component, the second dry powder comprises the protein, and wherein the composition further comprises a third dry powder comprising the crosslinking initiator; or wherein the first dry powder comprises the first component and the second dry powder comprises the second component; or wherein the first dry powder comprises the first component and the second dry powder comprises the second component and is in the form of a powder mixture.

5. The composition of any one of claims 3 or 4, wherein the dry powder mixture comprises the first dry powder in an amount of greater than or equal to 15 wt.% by mass and less than or equal to 40 wt.% by mass; or wherein the dry powder mixture comprises the second dry powder in an amount of greater than or equal to 20 wt.% by mass and less than or equal to 60 wt.% by mass.

6. The composition of any one of claims 1-5, wherein the composition comprises an active agent, optionally wherein the active agent is thrombin and/or another biologic agent, or wherein the active agent is crosslinked gelatin or starch particles or wherein the active agent is an antimicrobial agent.

7. The composition of any one of claims 1-6, wherein the hemostatic hydrogel has a burst pressure of greater than or equal to 10 mm Hg to less than or equal to less than or equal to 400 mm Hg.

8. The composition of any one of claims 1-7, wherein the composition comprises the crosslinking initiator in an amount of greater than or equal to 1 wt.% by mass and less than or equal to 30 wt.% by mass, optionally wherein the composition comprises the crosslinking initiator in an amount of greater than or equal to 15 wt.% by mass and less than or equal to 30 wt.% by mass.

9. The composition of any one of claims 1-8, wherein the composition has a measured crosslink time in whole blood and normal saline of greater than or equal to 15 seconds and less than or equal to 300 seconds and/or wherein the composition has a measured crosslink time of greater than or equal to 15 seconds and less than or equal to 150 seconds when the composition is added to whole blood and/or wherein the composition has a measured crosslink time of greater than or equal to 15 seconds and less than or equal to 250 seconds when the composition is added to normal saline.

10. The composition of any one of claims 1-9, wherein the protein consists essentially of particles having a particle size of greater than or equal to 50 microns and less than or equal to 500 microns, optionally wherein the protein consists essentially of particles having a particle size of greater than or equal to 100 microns and less than or equal to 250 microns.

11. The composition of any one of claims 1-10, wherein the protein comprises a plurality of particles having a tapped particle density of greater than or equal to 0.30 g/ml, optionally wherein the protein comprises a plurality of particles having a tapped particle density of greater than or equal to 0.50 g/ml.

12. The composition of any one claims 1-11, wherein the protein consists essentially of particles having a particle size of greater than or equal to 100 microns and less than or equal to 250 microns, and the composition has a measured crosslink time of greater than or equal to 15 seconds and less than or equal to 75 seconds when the composition is applied to whole blood; and/or wherein the protein consists essentially of particles having a particle size of greater than or equal to 100 microns and less than or equal to 250 microns, and the composition has a measured crosslink time of greater than or equal to 15 seconds and less than or equal to 100 seconds when the composition is added to normal saline; and/or wherein the protein comprises a plurality of particles having a tapped particle density of greater than or equal to 0.30 g/ml, and the composition has a measured crosslink time of greater than or equal to 15 seconds and less than or equal to 75 seconds when the composition is applied to whole blood; and/or wherein the protein comprises a plurality of particles having a tapped particle density of greater than or equal to 0.30 g/ml, and the composition has a measured crosslink time of greater than or equal to 15 seconds and less than or equal to 100 seconds when the composition is added to normal saline.

13. The composition of any one of claims 1-12, wherein the protein is roller compacted and/or granulated with the crosslinking initiator or wherein the protein is spray coated onto and/or over the crosslinking initiator.

14. The composition of any one of claims 1 to 13, for use in a method of controlling bleeding, the method comprising:
applying a crosslinkable dry powder composition to a bleeding/wound site,
allowing the dry powder composition to crosslink into a hemostatic hydrogel upon exposure to the bleeding/wound site capable of stopping or reducing bleeding at the bleeding/wound site.

## Patentansprüche

1. Trockene, pulverförmige, vernetzende hämostatische Zusammensetzung, umfassend:
eine erste Komponente, umfassend eine difunktionalisierte Polyalkylenoxid-basierte Komponente einer der Formen: 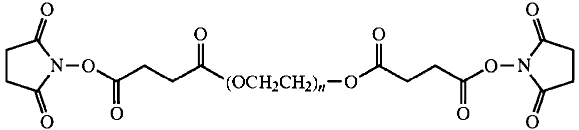 Poly(ethylenglycol)disuccinimidoylsuccinat 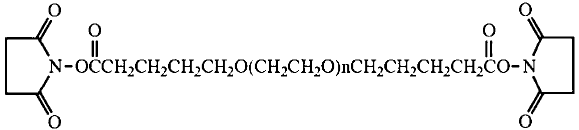 PEG-Disuccinimidylvalerat 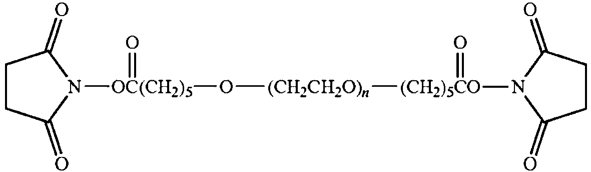 PEG-Disuccinimidylhexanoat; und
eine zweite Komponente, umfassend:
ein Protein, das zur Vernetzung mit der ersten Komponente in der Lage ist; und
einen Vernetzungsinitiator, der die Vernetzung der ersten Komponente mit dem Protein initiiert, wobei der Vernetzungsinitiator eine Base und/oder einen basischen Puffer umfasst, wobei die Base und/oder der basische Puffer Natriumbicarbonat und/oder eine Base, die keine Aminfunktionalität enthält, umfasst;
wobei bei Kontakt mit einer wässrigen Flüssigkeit die Vernetzung initiiert wird, um ein hämostatisches Hydrogel zu bilden.

2. Zusammensetzung gemäß Anspruch 1, wobei das Protein aus der Gruppe, bestehend aus: Human-Serumalbumin, rekombinantem Humanalbumin und Albumin tierischen Ursprungs, ausgewählt ist.

3. Zusammensetzung gemäß einem der Ansprüche 1 oder 2, wobei die Zusammensetzung mindestens ein erstes Trockenpulver und ein zweites Trockenpulver umfasst.

4. Zusammensetzung gemäß Anspruch 3, wobei das erste Trockenpulver die erste Komponente umfasst, das zweite Trockenpulver das Protein umfasst und wobei die Zusammensetzung ferner ein drittes Trockenpulver umfasst, das den Vernetzungsinitiator enthält; oder wobei das erste Trockenpulver die erste Komponente umfasst und das zweite Trockenpulver die zweite Komponente umfasst; oder wobei das erste Trockenpulver die erste Komponente umfasst und das zweite Trockenpulver die zweite Komponente umfasst und in Form einer Pulvermischung vorliegt.

5. Zusammensetzung gemäß einem der Ansprüche 3 oder 4, wobei die Trockenpulvermischung das erste Trockenpulver in einer Menge von größer oder gleich 15 Massen-% und kleiner oder gleich 40 Massen-% umfasst; oder wobei die Trockenpulvermischung das zweite Trockenpulver in einer Menge von größer oder gleich 20 Massen-% und kleiner oder gleich 60 Massen-% umfasst.

6. Zusammensetzung gemäß einem der Ansprüche 1-5, wobei die Zusammensetzung einen Wirkstoff umfasst, wobei der Wirkstoff optional Thrombin und/oder ein anderes biologisches Mittel ist, oder wobei der Wirkstoff vernetzte Gelatine- oder Stärkepartikel ist, oder wobei der Wirkstoff ein antimikrobielles Mittel ist.

7. Zusammensetzung gemäß einem der Ansprüche 1-6, wobei das hämostatische Hydrogel einen Berstdruck von größer oder gleich 10 mm Hg bis kleiner oder gleich bis kleiner oder gleich 400 mm Hg aufweist.

8. Zusammensetzung gemäß einem der Ansprüche 1-7, wobei die Zusammensetzung den Vernetzungsinitiator in einer Menge von größer oder gleich 1 Massen-% und kleiner oder gleich 30 Massen-% umfasst, wobei die Zusammensetzung optional den Vernetzungsinitiator in einer Menge von größer oder gleich 15 Massen-% und kleiner oder gleich 30 Massen-% umfasst.

9. Zusammensetzung gemäß einem der Ansprüche 1-8, wobei die Zusammensetzung eine gemessene Vernetzungszeit in Vollblut und normaler Kochsalzlösung von größer oder gleich 15 Sekunden und kleiner oder gleich 300 Sekunden aufweist und/oder wobei die Zusammensetzung eine gemessene Vernetzungszeit von größer oder gleich 15 Sekunden und kleiner oder gleich 150 Sekunden aufweist, wenn die Zusammensetzung zu Vollblut gegeben wird, und/oder wobei die Zusammensetzung eine gemessene Vernetzungszeit von größer oder gleich 15 Sekunden und kleiner oder gleich 250 Sekunden aufweist, wenn die Zusammensetzung zu normaler Kochsalzlösung gegeben wird.

10. Zusammensetzung gemäß einem der Ansprüche 1-9, wobei das Protein im Wesentlichen aus Partikeln mit einer Partikelgröße von größer oder gleich 50 Mikrometern und kleiner oder gleich 500 Mikrometern besteht, wobei das Protein optional im Wesentlichen aus Partikeln mit einer Partikelgröße von größer oder gleich 100 Mikrometern und kleiner oder gleich 250 Mikrometern besteht.

11. Zusammensetzung gemäß einem der Ansprüche 1-10, wobei das Protein eine Vielzahl von Partikeln mit einer Klopfpartikeldichte von größer oder gleich 0,30 g/ml umfasst, wobei das Protein optional eine Vielzahl von Partikeln mit einer Klopfpartikeldichte von größer oder gleich 0,50 g/ml umfasst.

12. Zusammensetzung gemäß einem der Ansprüche 1-11, wobei das Protein im Wesentlichen aus Partikeln mit einer Partikelgröße von größer oder gleich 100 Mikrometern und kleiner oder gleich 250 Mikrometern besteht und die Zusammensetzung eine gemessene Vernetzungszeit von größer oder gleich 15 Sekunden und kleiner oder gleich 75 Sekunden aufweist, wenn die Zusammensetzung auf Vollblut angewandt wird; und/oder wobei das Protein im Wesentlichen aus Partikeln mit einer Partikelgröße von größer oder gleich 100 Mikrometern und kleiner oder gleich 250 Mikrometern besteht und die Zusammensetzung eine gemessene Vernetzungszeit von größer oder gleich 15 Sekunden und kleiner oder gleich 100 Sekunden aufweist, wenn die Zusammensetzung zu normaler Kochsalzlösung gegeben wird; und/oder wobei das Protein eine Vielzahl von Partikeln mit einer Klopfpartikeldichte von größer oder gleich 0,30 g/ml umfasst und die Zusammensetzung eine gemessene Vernetzungszeit von größer oder gleich 15 Sekunden und kleiner oder gleich 75 Sekunden aufweist, wenn die Zusammensetzung auf Vollblut angewandt wird; und/oder wobei das Protein eine Vielzahl von Partikeln mit einer Klopfpartikeldichte von größer oder gleich 0,30 g/ml umfasst und die Zusammensetzung eine gemessene Vernetzungszeit von größer oder gleich 15 Sekunden und kleiner oder gleich 100 Sekunden aufweist, wenn die Zusammensetzung zu normaler Kochsalzlösung gegeben wird.

13. Zusammensetzung gemäß einem der Ansprüche 1-12, wobei das Protein mit dem Vernetzungsinitiator walzenverdichtet und/oder granuliert ist oder wobei das Protein auf den und/oder über den Vernetzungsinitiator gesprüht ist.

14. Zusammensetzung gemäß einem der Ansprüche 1 bis 13 zur Verwendung in einem Verfahren zur Blutstillung, wobei das Verfahren umfasst:
das Aufbringen einer vernetzbaren Trockenpulverzusammensetzung auf eine Blutungs-/Wundstelle,
das Vernetzen der Trockenpulverzusammensetzung zu einem hämostatischen Hydrogel bei Kontakt mit der Blutungs-/Wundstelle, das in der Lage ist, die Blutung an der Blutungs-/Wundstelle zu stoppen oder zu verringern.

## Revendications

1. Composition hémostatique pulvérulente sèche réticulante, comprenant :
un premier composant comprenant un composant à base de poly(oxyde d'alkylène) difonctionnel selon l'une quelconque des formes : 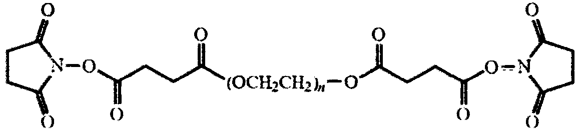 succinate de poly(éthylène glycol) disuccinimidoyle 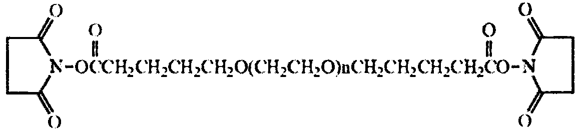 valérate de PEG-disuccinimidyle 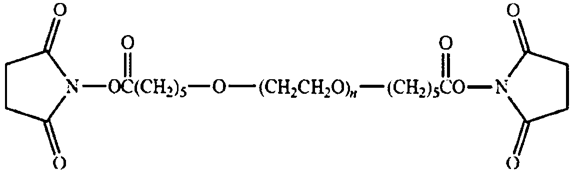 hexanoate de PEG-disuccinimidyle ; et
un deuxième composant comprenant :
une protéine qui est apte à réticuler avec le premier composant ; et
un initiateur de réticulation qui initie la réticulation du premier composant avec la protéine, dans lequel l'initiateur de réticulation comprend une base et/ou un tampon basique, dans lequel la base et/ou le tampon basique comprend du bicarbonate de sodium et/ou une base qui n'inclut pas de fonctionnalité amine ;
dans laquelle, lors d'une exposition à un liquide aqueux, la réticulation est initiée afin de former un hydrogel hémostatique.

2. Composition selon la revendication 1, dans laquelle la protéine est sélectionnée parmi le groupe consistant en : l'albumine sérique humaine, l'albumine humaine recombinante, et l'albumine d'origine animale.

3. Composition selon l'une quelconque des revendications 1 ou 2, dans laquelle la composition comprend au moins une première poudre sèche et une deuxième poudre sèche.

4. Composition selon la revendication 3, dans laquelle la première poudre sèche comprend le premier composant, la deuxième poudre sèche comprend la protéine, et dans laquelle la composition comprend en outre une troisième poudre sèche comprenant l'initiateur de réticulation ; ou dans laquelle la première poudre sèche comprend le premier composant et la deuxième poudre sèche comprend le deuxième composant ; ou dans laquelle la première poudre sèche comprend le premier composant et la deuxième poudre sèche comprend le deuxième composant et est sous la forme d'un mélange de poudres.

5. Composition selon l'une quelconque des revendications 3 ou 4, dans laquelle le mélange de poudre sèche comprend la première poudre sèche dans une quantité supérieure ou égale à 15 % en poids en masse et inférieure ou égale à 40 % en poids en masse ; ou dans laquelle le mélange de poudre sèche comprend la deuxième poudre sèche dans une quantité supérieure ou égale à 20 % en poids en masse et inférieure ou égale à 60 % en poids en masse.

6. Composition selon l'une quelconque des revendications 1 à 5, dans laquelle la composition comprend un agent actif, facultativement dans laquelle l'agent actif est de la thrombine et/ou un autre agent biologique, ou dans laquelle l'agent actif est de la gélatine réticulée ou des particules d'amidon ou dans laquelle l'agent actif est un agent antimicrobien.

7. Composition selon l'une quelconque des revendications 1 à 6, dans laquelle l'hydrogel hémostatique présente une pression d'éclatement supérieure ou égale à 10 mm Hg et inférieure ou égale à 400 mm Hg.

8. Composition selon l'une quelconque des revendications 1 à 7, dans laquelle la composition comprend l'initiateur de réticulation dans une quantité supérieure ou égale à 1 % en poids en masse et inférieure ou égale à 30 % en poids en masse, facultativement dans laquelle la composition comprend l'initiateur de réticulation dans une quantité supérieure ou égale à 15 % en poids en masse et inférieure ou égale à 30 % en poids en masse.

9. Composition selon l'une quelconque des revendications 1 à 8, dans laquelle la composition présente un temps de réticulation mesuré dans du sang total et dans une solution saline normale supérieur ou égal à 15 secondes et inférieur ou égal à 300 secondes et/ou dans laquelle la composition présente un temps de réticulation mesuré supérieur ou égal à 15 secondes et inférieur ou égal à 150 secondes lorsque la composition est ajoutée à du sang total et/ou dans laquelle la composition présente un temps de réticulation mesuré supérieur ou égal à 15 secondes et inférieur ou égal à 250 secondes lorsque la composition est ajoutée à une solution saline normale.

10. Composition selon l'une quelconque des revendications 1 à 9, dans laquelle la protéine se compose essentiellement de particules présentant une taille de particule supérieure ou égale à 50 microns et inférieure ou égale à 500 microns, facultativement dans laquelle la protéine se compose essentiellement de particules présentant une taille de particule supérieure ou égale à 100 microns et inférieure ou égale à 250 microns.

11. Composition selon l'une quelconque des revendications 1 à 10, dans laquelle la protéine comprend une pluralité de particules présentant une densité de particules après tassement supérieure ou égale à 0,30 g/ml, facultativement dans laquelle la protéine comprend une pluralité de particules présentant une densité de particules après tassement supérieure ou égale à 0,50 g/ml.

12. Composition selon l'une quelconque des revendications 1 à 11, dans laquelle la protéine se compose essentiellement de particules présentant une taille de particule supérieure ou égale à 100 microns et inférieure ou égale à 250 microns, et la composition présente un temps de réticulation mesuré supérieur ou égal à 15 secondes et inférieur ou égal à 75 secondes lorsque la composition est appliquée à du sang total ; et/ou dans laquelle la protéine se compose essentiellement de particules présentant une taille de particule supérieure ou égale à 100 microns et inférieure ou égale à 250 microns, et la composition présente un temps de réticulation mesuré supérieur ou égal à 15 secondes et inférieur ou égal à 100 secondes lorsque la composition est ajoutée à une solution saline normale ;
et/ou dans laquelle la protéine comprend une pluralité de particules présentant une densité de particules après tassement supérieure ou égale à 0,30 g/ml, et la composition présente un temps de réticulation mesuré supérieur ou égal à 15 secondes et inférieur ou égal à 75 secondes lorsque la composition est appliquée à du sang total ; et/ou dans laquelle la protéine comprend une pluralité de particules présentant une densité de particules après tassement supérieure ou égale à 0,30 g/ml, et la composition présente un temps de réticulation mesuré supérieur ou égal à 15 secondes et inférieur ou égal à 100 secondes lorsque la composition est ajoutée à une solution saline normale.

13. Composition selon l'une quelconque des revendications 1 à 12, dans laquelle la protéine est compactée au rouleau et/ou granulée avec l'initiateur de réticulation ou dans laquelle la protéine est revêtue par pulvérisation sur et/ou par-dessus l'initiateur de réticulation.

14. Composition selon l'une quelconque des revendications 1 à 13, pour une utilisation dans un procédé de commande d'un saignement, le procédé comprenant :
appliquer une composition pulvérulente sèche réticulable sur un site de saignement/plaie,
laisser la composition pulvérulente sèche réticuler en un hydrogel hémostatique lors d'une exposition au site de saignement/plaie apte à arrêter ou à réduire le saignement au niveau du site de saignement/plaie.
